# EUROPEAN PATENT SPECIFICATION

(11) **EP 2 189 110 B1**
(45) Date of publication and mention of the grant of the patent: **23.02.2022**
(21) Application number: 08830945.5
(22) Date of filing: 09.09.2008
(51) Int. Cl.: A61B 3/12, A61B 3/14, A61B 3/00

(54) **EYEGROUND OBSERVING DEVICE, EYEGROUND IMAGE PROCESSING DEVICE, AND PROGRAM**
VORRICHTUNG ZUR BEOBACHTUNG DES AUGENGRUNDES, AUGENGRUNDBILDVERARBEITUNGSVORRICHTUNG UND PROGRAMM
DISPOSITIF D'OBSERVATION DU FOND DE L'OEIL, DISPOSITIF ET PROGRAMME DE TRAITEMENT D'IMAGES DU FOND DE L'OEIL

(30) Priority: 10.09.2007 JP 2007233703
(43) Date of publication of application: 26.05.2010
(73) Proprietor: The University of Tokyo, Bunkyo-ku, Tokyo 113-8654 (JP); Kabushiki Kaisha TOPCON, Tokyo 174-8580 (JP)
(72) Inventor: TOMIDOKORO, Atsuo, Tokyo 113-8654 (JP); KONNO, Shinsuke, Tokyo 113-8654 (JP); ARAIE, Makoto, Tokyo 113-8654 (JP); AOKI, Hiroyuki, Tokyo 174-8580 (JP); FUJIMURA, Takashi, Tokyo 174-8580 (JP); KIKAWA, Tsutomu, Tokyo 174-8580 (JP)
(74) Representative: Gagel, Roland
(86) International application number: PCT/JP2008/002481
(87) International publication number: WO 2009/034705

(56) References cited:
- WO-A1-2006/022045
- JP-A- 2007 130 403
- JP-A- 2007 185 243
- JP-A- 2007 185 244
- JP-A- 2007 325 831
- JP-A- 2008 073 099
- US-A1- 2006 119 858
- US-A1- 2007 195 269

## Description

### TECHNICAL FIELD

The present invention relates to a fundus oculi observation device for observing the fundus oculi, a fundus oculi image processing device that processes an image of the fundus oculi, and a program.

### BACKGROUND ART

In recent years, the OCT (Optical Coherence Tomography) technique of forming an image representing the surface morphology or internal morphology of a measured object by using a light beam from a laser light source or the like has received attention. Unlike an X-ray CT device, the OCT technique does not have invasiveness to a human body, and therefore, is expected to be applied particularly in the medical field.

Patent Document 1 discloses a device (an optical image measurement device) having such a configuration that: a measuring arm scans an object by using a rotary deflection mirror (a Galvano mirror); a reference mirror is disposed to a reference arm; at the outlet thereof, such an interferometer is used that the intensity of a light appearing due to interference of light fluxes from the measuring arm and the reference arm is analyzed by a spectrometer; and the reference arm is provided with a device that gradually changes the light flux phase of the reference light in non-continuous values.

This optical image measurement device uses a method of the so-called "Fourier Domain OCT (Optical Coherence Tomography)." That is to say, the morphology in the depth direction (the z-direction) of a measured object is imaged by radiating a low-coherence light beam to the measured object, acquiring the spectrum intensity distribution of the reflected light, and executing a process such as Fourier transform thereon.

Furthermore, this optical image measurement device is provided with a Galvano mirror that scans with a light beam (a signal light) so as to be capable of forming an image of a desired measurement target region of a measured object. Because this optical image measurement device scans with the light beam only in one direction (the x-direction) orthogonal to the z-direction, a formed image is a two-dimensional tomographic image in the depth direction (the z-direction) along a scan direction of the light beam (the x-direction).

Patent Document 2 discloses a technique of scanning with a signal light in the horizontal direction and the vertical direction to form a plurality of two-dimensional tomographic images in the horizontal direction and, based on the plurality of tomographic images, acquiring and imaging three-dimensional tomographic information of a measurement range. Examples of a method for three-dimensional imaging is a method of arranging and displaying a plurality of tomographic images in the vertical direction (referred to as stack data or the like), and a method of forming a three-dimensional image by generating volume data from a plurality of tomographic images and executing a rendering process on this volume data.

Patent Document 3 discloses a configuration of applying the optical image measurement device as described above in the ophthalmologic field.

Patent Documents 4 and 5 disclose other types of optical image measurement devices. Patent Document 4 describes such a type of an optical image measurement device that changes the wavelength of a light radiated to a measured object. This optical image measurement device is called the Swept Source type, or the like.

Further, Patent Document 5 describes an optical image measurement device that radiates a light having a predetermined beam diameter to a measured object and forms an image in a cross section orthogonal to the travelling direction of the light. This optical image measurement device is called the full-field type, the en-face type, or the like.

Further, as a device that captures an image of the fundus oculi surface, a retinal camera is widely used (for example, refer to Patent Document 6).

Further, Patent Document 7 discloses a technique for anatomical mapping utilizing optical coherence tomography. A 3-D data set is acquired by SD-OCT, and the 3-D data set can be reduced to generate an ocular mapping. Further, partial segmentation is used to generate a partial fundus image. In addition, a fundus image is generated from intensity data included in the spectra obtained by SD-OCT.

[Patent Document 1] Japanese Unexamined Patent Application Publication No. 11-325849
[Patent Document 2] Japanese Unexamined Patent Application Publication No. 2002-139421
[Patent Document 3] Japanese Unexamined Patent Application Publication No. 2003-543
[Patent Document 4] Japanese Unexamined Patent Application Publication No. 2007-24677
[Patent Document 5] Japanese Unexamined Patent Application Publication No. 2006-153838
[Patent Document 6] Japanese Unexamined Patent Application Publication No. 2007-7454
[Patent Document 7] US Patent Application Publication No. US 2006/0119858 A1

### DISCLOSURE OF THE INVENTION

### PROBLEM THAT THE INVENTION IS TO SOLVE

In an image of the fundus oculi acquired by using the OCT technique, an image of a region just below a blood vessel (a just-below-blood-vessel region) is unclear due to the influence of a vascular wall, blood or blood flow. Therefore, for observation of a tomographic image of the fundus oculi, analysis of the thickness of the layer of the retina with reference to a tomographic image, and so on, it is desirable to accurately specify the vascular position in the image in order to increase the reliability of the observation and analysis.

However, it has been difficult to specify a vascular region in an OCT image with high accuracy by conventional techniques. Searching and specifying an unclear region in a tomographic image of the fundus oculi as a just-below-blood-vessel region and specifying a vascular region based on this just-below-blood-vessel region has been conventionally executed. However, it is difficult to specify the position of a blood vessel with high accuracy by this method in a case that only a totally unclear tomographic image can be obtained due to the influence of opacity of the eyeball caused by cataract or the like.

Moreover, it is difficult, only by analyzing the OCT image, to determine whether the unclear region in the image results from a blood vessel or other causes.

The present invention was made for solving the above problems, and an object of the present invention is to provide a fundus oculi observation device, a fundus oculi image processing device and a program, which are capable of increasing the accuracy of a process of specifying a vascular position in an OCT image of the fundus oculi.

### MEANS FOR SOLVING THE ABOVE PROBLEM

In order to achieve the abovementioned objects, in a first aspect of the present invention, a fundus oculi observation device comprises: an acquiring part configured to acquire a tomographic image of a fundus oculi and a two-dimensional image of a surface of the fundus oculi; a first specifying part configured to analyze the tomographic image to specify a vascular region in the tomographic image; a second specifying part configured to analyze the two-dimensional image to specify a vascular region in the two-dimensional image; an image processor configured to obtain a common region of the vascular region in the tomographic image and the vascular region in the two-dimensional image, and specify a region in the tomographic image corresponding to the common region; a display; and a controller configured to control the display to display the tomographic image so that the region corresponding to the common region can be visually recognized.

Further, in a second aspect of the present invention, in the fundus oculi observation device according to the first aspect, the acquiring part includes: a part configured to split a low-coherence light into a signal light and a reference light, superimpose the signal light propagated through the fundus oculi and the reference light propagated through a reference object to generate an interference light, and detect the interference light to form the tomographic image of the fundus oculi; and an imaging part configured to radiate an illumination light to the fundus oculi, and detect a fundus oculi reflected light of the illumination light to capture the two-dimensional image of the surface of the fundus oculi.

Further, in a third aspect of the present invention, in the fundus oculi observation device according to the first aspect, the acquiring part includes: a part configured to split a low-coherence light into a signal light and a reference light, superimpose the signal light propagated through the fundus oculi and the reference light propagated through a reference object to generate an interference light, and detect the interference light to form the tomographic image of the fundus oculi; and an accepting part configured to accept the two-dimensional image of the surface of the fundus oculi.

Further, in a fourth aspect of the present invention, in the fundus oculi observation device according to the first aspect, the acquiring part includes: an accepting part configured to accept the tomographic image of the fundus oculi; and an imaging part configured to radiate an illumination light to the fundus oculi, and detect a fundus oculi reflected light of the illumination light to capture the two-dimensional image of the surface of the fundus oculi.

Further, in a fifth aspect of the present invention, in the fundus oculi observation device according to the first aspect, the image processor is configured to erase an image of the region in the tomographic image corresponding to the common region.

Further, in a sixth aspect of the present invention, in the fundus oculi observation device according to the fifth aspect, wherein the image processor is configured to analyze the tomographic image to specify a layer position of the fundus oculi in a neighborhood region of the common region, and add an image representing the layer position to the region corresponding to the common region, based on the layer position in the neighborhood region.

Further, in a seventh aspect of the present invention, in the fundus oculi observation device according to the first aspect, the image processor is configured to analyze the tomographic image to specify a layer position of the fundus oculi in a neighborhood region of the common region, and add an image representing the layer position to the region in the tomographic image corresponding to the common region, based on the layer position in the neighborhood region.

Further, in an eighth aspect of the present invention, in the fundus oculi observation device according to the sixth aspect, the image processor is configured to specify a boundary region of a layer as the layer position based on pixel values of pixels in the neighborhood region, estimate a boundary position of the layer in the common region based on a morphology of the boundary region, and add an image representing the estimated boundary position as an image representing the layer position.

Further, in a ninth aspect of the present invention, in the fundus oculi observation device according to the seventh aspect, the image processor is configured to specify a boundary region of a layer as the layer position based on pixel values of pixels in the neighborhood region, estimate a boundary position of the layer in the common region based on a morphology of the boundary region, and add an image representing the estimated boundary position as an image representing the layer position.

Further, in a tenth aspect of the present invention, in the fundus oculi observation device according to the eighth aspect, the image processor is configured to, for each of the neighborhood regions on both sides of the common region, obtain a position of the boundary region of the layer at a boundary between the neighborhood region and the common region, estimate a position on a straight line connecting positions on both the sides as the boundary position, and add the line as the image representing the boundary position.

Further, in an eleventh aspect of the present invention, in the fundus oculi observation device according to the ninth aspect, the image processor is configured to, for each of the neighborhood regions on both sides of the common region, obtain a position of the boundary region of the layer at a boundary between the neighborhood region and the common region, estimate a position on a straight line connecting positions on both the sides as the boundary position, and add the line as the image representing the boundary position.

Further, in a twelfth aspect of the present invention, in the fundus oculi observation device according to the eighth aspect, the image processor is configured to, for each of the neighborhood regions on both sides of the common region, obtain a position and slope of the boundary region of the layer at a boundary between the neighborhood region and the common region, estimate a position on a spline curve connecting positions on both the sides as the boundary position based on the position and slope, and add the spline curve as an image representing the boundary position.

Further, in a thirteenth aspect of the present invention, in the fundus oculi observation device according to the ninth aspect, the image processor is configured to, for each of the neighborhood regions on both sides of the common region, obtain a position and slope of the boundary region of the layer at a boundary between the neighborhood region and the common region, estimate a position on a spline curve connecting positions on both the sides as the boundary position based on the position and slope, and add the spline curve as an image representing the boundary position.

Further, in a fourteenth aspect of the present invention, the fundus oculi observation device according to the sixth aspect comprises a calculator configured to calculate a layer thickness of the fundus oculi in the common region, based on the image representing the layer position.

Further, in a fifteenth aspect of the present invention, the fundus oculi observation device according to the seventh aspect comprises a calculator configured to calculate a layer thickness of the fundus oculi in the common region, based on the image representing the layer position.

Further, in a sixteenth aspect of the present invention, a fundus oculi observation method for analyzing a tomographic image of a fundus oculi and a two-dimensional image of a surface of the fundus oculi, comprising steps of: analyzing the tomographic image to specify a vascular region in the tomographic image; analyzing the two-dimensional image to specify a vascular region in the two-dimensional image; obtaining a common region of the vascular region in the tomographic image and the vascular region in the two-dimensional image; specifying a region in the tomographic image corresponding to the common region; and displaying the tomographic image so that the region corresponding to the common region can be visually recognized.

### EFFECT OF THE INVENTION

According to the present invention, it is possible to specify a vascular region in a tomographic image of the fundus oculi, specify a vascular region in a two-dimensional image of the fundus oculi surface, obtain a common region of the vascular region in the tomographic image and the vascular region in the two-dimensional image, specify a region in the tomographic image corresponding to the common region, and display the tomographic image so that the region corresponding to the common region can be visually recognized.

Thus, according to the present invention, it is possible to specify, of a vascular region in a tomographic image of the fundus oculi, a vascular region common to that in a two-dimensional image of the fundus oculi surface. Therefore, it is possible to specify the vascular region in the tomographic image with higher accuracy than before based on both the images. Moreover, since it is possible to display the tomographic image so that the region corresponding to the common region can be visually recognized, it is possible to present the position of the vascular region in the tomographic image with high accuracy.

### BRIEF DESCRIPTION OF THE DRAWINGS

Fig. 1 is a schematic configuration diagram showing an example of the entire configuration of an embodiment of a fundus oculi observation device according to the present invention.
Fig. 2 is a schematic configuration diagram showing an example of the configuration of a scan unit installed in a retinal camera unit in the embodiment of the fundus oculi observation device according to the present invention.
Fig. 3 is a schematic configuration diagram showing an example of the configuration of an OCT unit in the embodiment of the fundus oculi observation device according to the present invention.
Fig. 4 is a schematic block diagram showing an example of the hardware configuration of an arithmetic and control unit in the embodiment of the fundus oculi observation device according to the present invention.
Fig. 5 is a schematic block diagram showing an example of the configuration of a control system in the embodiment of the fundus oculi observation device according to the present invention.
Fig. 6 is a schematic block diagram showing an example of the configuration of a control system in the embodiment of the fundus oculi observation device according to the present invention.
Fig. 7 is a schematic view showing an example of the pattern of a tomographic image formed in the embodiment of the fundus oculi observation device according to the present invention.
Fig. 8 is a schematic explanation view for explaining an example of a process of specifying a vascular region in a tomographic image in the embodiment of the fundus oculi observation device according to the present invention.
Fig. 9 is a schematic view showing an example of the pattern of a fundus oculi image captured in the embodiment of the fundus oculi observation device according to the present invention.
Fig. 10 is a schematic explanation view for explaining an example of a process of estimating a layer position in the tomographic image in the embodiment of the fundus oculi observation device according to the present invention.
Fig. 11 is a schematic explanation view for explaining an example of a process of estimating a layer position in the tomographic image in the embodiment of the fundus oculi observation device according to the present invention.
Fig. 12 is a schematic explanation view for explaining an example of a process of estimating a layer position in the tomographic image in the embodiment of the fundus oculi observation device according to the present invention.
Figs. 13A and 13B are schematic views showing an example of a scan pattern of a signal light in the embodiment of the fundus oculi observation device according to the present invention. Fig. 13A shows an example of the scan pattern of the signal light when the fundus oculi is seen from the incident side of the signal light into an eye.
Moreover, Fig. 13B shows an example of an arrangement pattern of scan points on each scan line.
Fig. 14 is a schematic view showing an example of a scan pattern of the signal light and the pattern of a tomographic image formed along each scan line in the present embodiment of the fundus oculi observation device according to the present invention.
Fig. 15 is a flow chart showing an example of a usage pattern in the embodiment of the fundus oculi observation device according to the present invention.
Fig. 16 is a schematic block diagram showing an example of the configuration of an embodiment of a fundus oculi image processing device according to the present invention.

### DESCRIPTION OF REFERENCE NUMERALS AND SYMBOLS

- 1: fundus oculi observation device (optical image measurement device)
- 1A: retinal camera unit
- 141: scan unit
- 150: OCT unit
- 160: low-coherence light source
- 174: reference mirror
- 180: spectrometer
- 184: CCD
- 200: arithmetic and control unit
- 210: controller
- 211: main controller
- 220: image forming part
- 230: image processor
- 231: vascular region specifying part
- 232: tomographic image analyzer
- 233: fundus oculi image analyzer
- 234: tomographic image processor
- 235: common region specifying part
- 236: image eraser
- 237: layer position specifying part
- 238: image adder
- 239: layer thickness calculator
- 240: user interface
- 240A: display
- 240B: manipulation part

### BEST MODE FOR CARRYING OUT THE INVENTION

An example of an embodiment of a fundus oculi observation device, a fundus oculi image processing device and a program according to the present invention will be described in detail with reference to the drawings.

The fundus oculi observation device according to the present invention has a function of acquiring a tomographic image of the fundus oculi and/or a function of capturing a two-dimensional image of the fundus oculi surface. The former function can be realized by an arbitrary OCT technique such as the Fourier Domain type, the Swept Source type and the full-field type. The latter function can be realized by the same configuration as that of a retinal camera, for example.

Such a configuration acts as an example of the "acquiring part" of the present invention.

In the case of having only the function of acquiring a tomographic image of the fundus oculi, the fundus oculi observation device according to the present invention has a function of accepting a two-dimensional image of the fundus oculi image captured by an external device. On the other hand, in the case of having only the function of acquiring a two-dimensional image of the fundus oculi surface, the fundus oculi observation device according to the present invention has a function of accepting a tomographic image of the fundus oculi acquired by an external device. Such a function of accepting an image can be realized by a configuration to control data communication with an external device, or a configuration to read an image from a recording medium in which the image is recorded.

Below, a fundus oculi observation device configured to be capable of acquiring both a tomographic image of the fundus oculi and an image of the surface will be described, and thereafter, a fundus oculi observation device having another configuration will be described. Furthermore, after the description of the fundus oculi observation device, the fundus oculi image processing device and program according to the present invention will be described.

### [DEVICE CONFIGURATION]

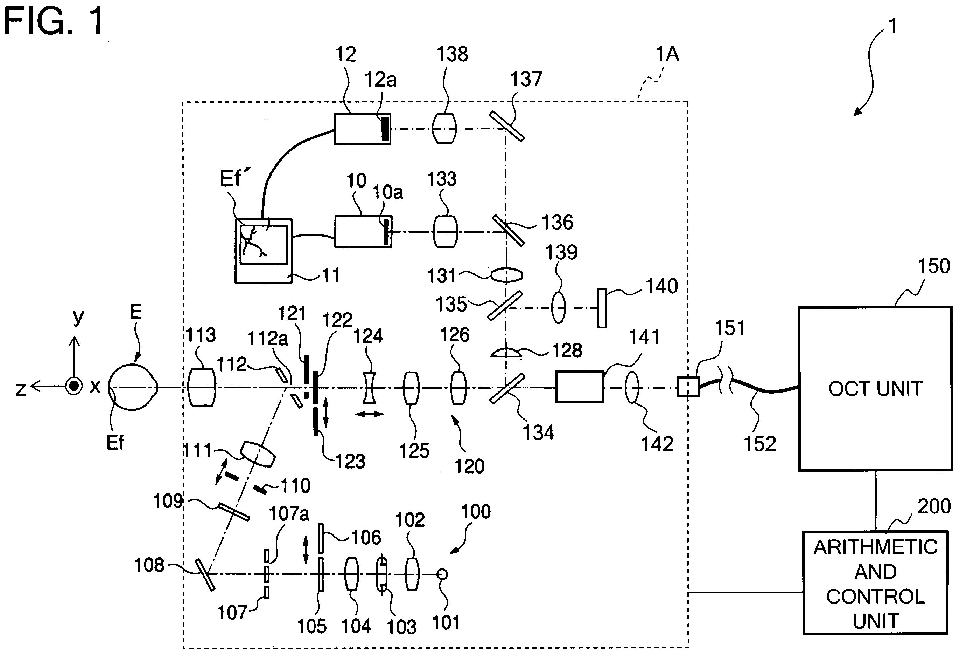

A fundus oculi observation device 1 shown in Fig. 1 captures a two-dimensional image of the fundus oculi surface by the same configuration as that of a conventional retinal camera, and also acquires an OCT image of the fundus oculi by the Fourier-Domain-type OCT technique.

### [ENTIRE CONFIGURATION]

As shown in Fig. 1, the fundus oculi observation device 1 includes a retinal camera unit 1A, an OCT unit 150, and an arithmetic and control unit 200. The retinal camera unit 1A has an optical system almost the same as that of a conventional retinal camera. The OCT unit 150 houses an optical system for acquiring an OCT image. The arithmetic and control unit 200 executes various arithmetic processes and control processes, in addition to a process of forming an OCT image based on data obtained by the OCT unit 150.

To the OCT unit 150, one end of a connection line 152 is attached. The other end of the connection line 152 is connected to the retinal camera unit 1A by a connector part 151. An optical fiber runs through inside the connection line 152. Thus, the OCT unit 150 and the retinal camera unit 1A are optically connected via the connection line 152.

### [Configuration of Retinal Camera Unit]

The retinal camera unit 1A has an optical system for forming a two-dimensional image of the fundus oculi surface. Here, a two-dimensional image of the fundus oculi surface represents images obtained by imaging the fundus oculi surface such as a color image, a monochrome image and a fluorescent image (a fluorescein angiography image, an indocyanine green fluorescent image, and so on). As is a conventional retinal camera, the retinal camera unit 1A is provided with an illumination optical system 100 that illuminates a fundus oculi Ef, and an imaging optical system 120 that leads the fundus oculi reflected light of the illumination light to an imaging device 10.

The illumination optical system 100 includes an observation light source 101, a condenser lens 102, an imaging light source 103, a condenser lens 104, exciter filters 105 and 106, a ring transparent plate 107, a mirror 108, an LCD (Liquid Crystal Display) 109, an illumination diaphragm 110, a relay lens 111, an aperture mirror 112, and an objective lens 113.

The observation light source 101 outputs an illumination light having a wavelength of visible region included in the range of about 400-700 nm, for example. The imaging light source 103 outputs an illumination light having a wavelength of near-infrared region included in the range of about 700-800 nm, for example. This near-infrared light is set so as to have a shorter wavelength than a light used by the OCT unit 150 (described later).

Further, the imaging optical system 120 includes the objective lens 113, (an aperture 112a of) the aperture mirror 112, an imaging diaphragm 121, barrier filters 122 and 123, a magnifying lens 124, a relay lens 125, an imaging lens 126, a dichroic mirror 134, a field lens 128, a half mirror 135, a relay lens 131, a dichroic mirror 136, an imaging lens 133, the imaging device 10 (an image pick-up element 10a), a reflection mirror 137, an imaging lens 138, the imaging device 12 (an image pick-up element 12a), a lens 139, and an LCD 140.

Furthermore, the imaging optical system 120 is provided with the dichroic mirror 134, the half mirror 135, the dichroic mirror 136, the reflection mirror 137, the imaging lens 138, the lens 139, and the LCD 140.

The dichroic mirror 134 reflects the fundus oculi reflected light of the illumination light coming from the illumination optical system 100, and transmits a signal light LS coming from the OCT unit 150.

Further, the dichroic mirror 136 transmits the fundus oculi reflected light of the illumination light coming from the observation light source 101, and reflects the fundus oculi reflected light of the illumination light coming from the imaging light source 103.

The LCD 140 displays a fixation target (an internal fixation target) for fixing an eye E. After focused by the lens 139, the light from the LCD 140 is reflected by the half mirror 135, propagated through the field lens 128, and reflected by the dichroic mirror 134.

Furthermore, this light is propagated through the imaging lens 126, the relay lens 125, the magnifying lens 124, the (aperture 112a of the) aperture mirror 112, the objective lens 113 and so on, and enters the eye E. Consequently, an internal fixation target is projected onto the fundus oculi Ef of the eye E.

The image pick-up element 10a is an image pick-up element such as a CCD (Charge Coupled Device) or a CMOS (Complementary Metal Oxide Semiconductor). The image pick-up element 10a specifically detects a light having a wavelength of near-infrared region. In other words, the imaging device 10 functions as an infrared TV camera that detects a near-infrared light. The imaging device 10 outputs a video signal as the result of detection of the near-infrared light. For imaging by the imaging device 10, the illumination light from the imaging light source 103 is used, for example.

A touch panel monitor 11 displays a two-dimensional image (a fundus oculi image Ef') of the surface of the fundus oculi Ef based on the video signal. Moreover, this video signal is transmitted to the arithmetic and control unit 200.

The image pick-up element 12a is an image pick-up element such as a CCD or a CMOS. The image pick-up element 12a specifically detects a light having a wavelength of visible region. In other words, the imaging device 12 is a TV camera that detects a visible light. The imaging device 12 outputs a video signal as the result of detection of the visible light. For fundus oculi imaging by the imaging device 12, the illumination light from the observation light source 101 is used, for example.

The touch panel monitor 11 displays a two-dimensional image (the fundus oculi image Ef') of the surface of the fundus oculi Ef based on the video signal. Moreover, this video signal is sent to the arithmetic and control unit 200.

The retinal camera unit 1A is provided with a scan unit 141 and a lens 142. The scan unit 141 scans a target position on the fundus oculi Ef of a light (the signal light LS; described later) outputted from the OCT unit 150.

The lens 142 collimates the signal light LS led from the OCT unit 150 through the connection line 152, and makes the light enter the scan unit 141. Further, the lens 142 focuses the fundus oculi reflected light of the signal light LS propagated through the scan unit 141.

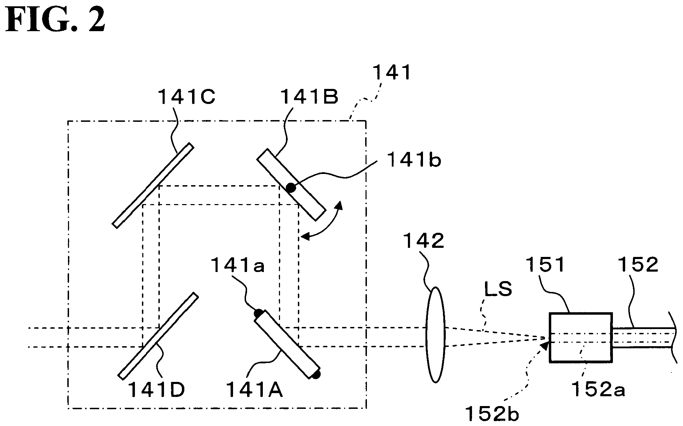

Fig. 2 shows an example of the configuration of the scan unit 141. The scan unit 141 includes Galvano mirrors 141A and 141B, and reflection mirrors 141C and 141D.

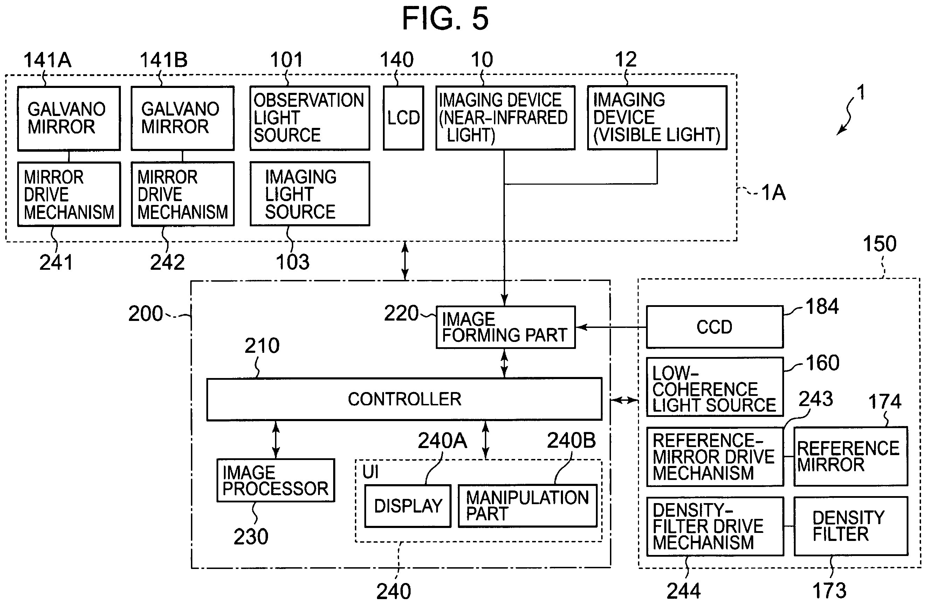

The Galvano mirrors 141A and 141B are reflection mirrors arranged so as to be rotatable about rotary shafts 141a and 141b, respectively. The respective Galvano mirrors 141A and 141B are rotated about the rotary shafts 141a and 141b by drive mechanisms described later (mirror drive mechanisms 241 and 242 shown in Fig. 5).

Consequently, the directions of the reflection surfaces (surfaces to reflect the signal light LS) of the respective Galvano mirrors 141A and 141B are changed.

The rotary shafts 141a and 141b are arranged orthogonally to each other. In Fig. 2, the rotary shaft 141a of the Galvano mirror 141A is arranged in the parallel direction to the paper surface. On the other hand, the rotary shaft 141b of the Galvano mirror 141B is arranged in the orthogonal direction to the paper surface.

That is to say, the Galvano mirror 141B is configured to be rotatable in the direction indicated by an arrow pointing to both directions in Fig. 2, whereas the Galvano mirror 141A is configured to be rotatable in the direction orthogonal to the arrow pointing to both the directions. Consequently, the Galvano mirrors 141A and 141B act to change the reflection directions of the signal light LS into directions orthogonal to each other, respectively. As apparent from Figs. 1 and 2, a scan with the signal light LS is performed in the x-direction when the Galvano mirror 141A is rotated, and a scan with the signal light LS is performed in the y-direction when the Galvano mirror 141B is rotated.

The signal light LS reflected by the Galvano mirrors 141A and 141B is reflected by the reflection mirrors 141C and 141D, and travels in the same direction as having entered the Galvano mirror 141A.

An end surface 152b of an optical fiber 152a inside the connection line 152 is arranged so as to face the lens 142. The signal light LS emitted from the end surface 152b travels while expanding the beam diameter thereof toward the lens 142, and is collimated by the lens 142. On the contrary, the signal light LS propagated through the fundus oculi Ef is focused to the end surface 152b by the lens 142, and enters the optical fiber 152a.

### [Configuration of OCT Unit]

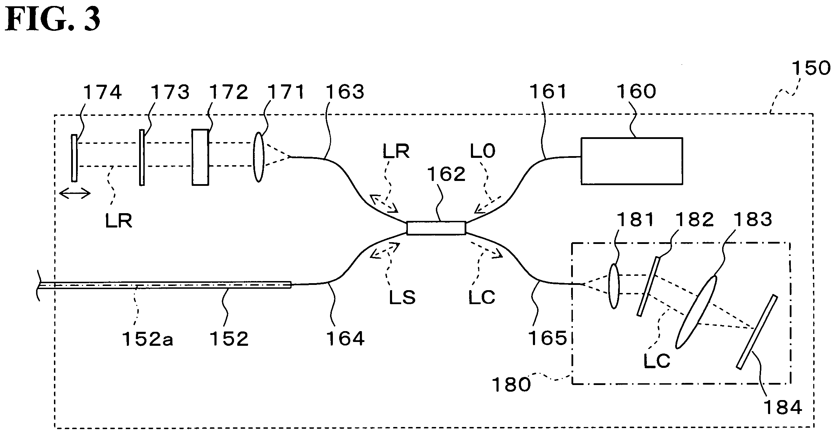

Next, the configuration of the OCT unit 150 will be described with reference to Fig. 3. The OCT unit 150 has an optical system for forming an OCT image of the fundus oculi.

The OCT unit 150 is provided with an optical system almost the same as that of a conventional optical image measurement device. That is to say, the OCT unit 150 splits a low-coherence light into a reference light and a signal light, superimposes the signal light propagated through an eye and the reference light propagated through a reference object to generate an interference light, and detects this interference light. This detection result (a detection signal) is inputted into the arithmetic and control unit 200. The arithmetic and control unit 200 analyzes this detection signal and forms a tomographic image or three-dimensional image of the fundus oculi.

A low-coherence light source 160 is composed of a broadband light source that outputs a low-coherence light L0. For example, a super luminescent diode (SLD), a light emitting diode (LED) or the like is used as the broadband light source.

The low-coherence light L0 is, for example, a light that includes a light having a wavelength of near-infrared region and that has a temporal coherence length of about several tens of micrometers. The low-coherence light L0 has a longer wavelength than the illumination light (having a wavelength of about 400-800 nm) of the retinal camera unit 1A, for example, a wavelength included in the range of about 800-900 nm.

The low-coherence light L0 outputted from the low-coherence light source 160 is led to an optical coupler 162 through an optical fiber 161. The optical fiber 161 is composed of, for example, a single mode fiber, a PM (polarization maintaining) fiber or the like. The optical coupler 162 splits the low-coherence light L0 into a reference light LR and the signal light LS.

The optical coupler 162 acts as both a part for splitting a light (a splitter) and a part for superposing lights (a coupler), but will be herein referred to as an "optical coupler" idiomatically.

The reference light LR generated by the optical coupler 162 is led by an optical fiber 163 composed of a single mode fiber or the like, and is emitted from the end surface of the fiber. Furthermore, after collimated by a collimator lens 171, the reference light LR is propagated through a glass block 172 and a density filter 173, and reflected by a reference mirror 174. The reference mirror 174 is an example of the "reference object" of the present invention.

The reference light LR reflected by the reference mirror 174 is again propagated through the density filter 173 and the glass block 172, focused to the fiber end surface of the optical fiber 163 by the collimator lens 171, and led to the optical coupler 162 through the optical fiber 163.

Here, the glass block 172 and the density filter 173 act as a delaying part for matching the optical path lengths (the optical distances) of the reference light LR and the signal light LS, and also as a dispersion compensating part for matching the dispersion properties of the reference light LR and the signal light LS.

Further, the density filter 173 also acts as a neutral density filter that reduces the light amount of the reference light LR. The density filter 173 is composed of, for example, a rotary-type ND (Neutral Density) filter. The density filter 173 is driven to rotate by a drive mechanism (a density-filter drive mechanism 244 shown in Fig. 5) including a driver such as a motor. Consequently, the light amount of the reference light LR contributing to generation of the interference light LD is changed.

Further, the reference mirror 174 is configured to be movable in the traveling direction of the reference light LR (a direction of an arrow pointing to both sides shown in Fig. 3). Thus, it is possible to ensure an optical path length of the reference light LR according to the axial length of the eye E, a working distance (a distance between the objective lens 113 and the eye E), and so on. Moreover, by moving the reference mirror 174, it is possible to acquire an image at an arbitrary depth position of the fundus oculi Ef. The reference mirror 174 is moved by a drive mechanism (a reference-mirror drive mechanism 243 shown in Fig. 5) including a driver such as a motor.

On the other hand, the signal light LS generated by the optical coupler 162 is led to the end part of the connection line 152 through an optical fiber 164 composed of a single mode fiber or the like. The optical fiber 152a runs through inside the connection line 152. Here, the optical fiber 164 and the optical fiber 152a may be composed of a single optical fiber, or may be integrally formed by joining the end surfaces of the respective fibers, for example. Anyway, it is sufficient as far as the optical fibers 164 and 152a are configured to be capable of transmitting the signal light LS between the retinal camera unit 1A and the OCT unit 150.

The signal light LS is led through the inside of the connection line 152 and guided to the retinal camera unit 1A. Furthermore, the signal light LS is propagated through the lens 142, the scan unit 141, the dichroic mirror 134, the imaging lens 126, the relay lens 125, the magnifying lens 124, the imaging diaphragm 121, the aperture 112a of the aperture mirror 112 and the objective lens 113, and radiated to the eye E. For radiating the signal light LS to the eye E, the barrier filters 122 and 123 are previously retracted from the optical path, respectively.

The signal light LS having entered the eye E is formed into an image on the fundus oculi Ef and then reflected. At this moment, the signal light LS not only is reflected by the surface of the fundus oculi Ef but also reaches a deep region of the fundus oculi Ef to be scattered at the refractive index boundary. Therefore, the signal light LS propagated through the fundus oculi Ef contains information reflecting the surface morphology of the fundus oculi Ef and information reflecting the state of backscatter at the refractive index boundary of deep layer tissues of the fundus oculi Ef.

The fundus oculi reflected light of the signal light LS travels reversely on the abovementioned path within the retinal camera unit 1A to be focused to the end surface 152b of the optical fiber 152a, enters the OCT unit 150 through the optical fiber 152a, and returns to the optical coupler 162 through the optical fiber 164.

The optical coupler 162 superimposes the signal light LS having returned through the eye E and the reference light LR reflected by the reference mirror 174 to generate an interference light LC. This interference light LC is led to a spectrometer 180 through an optical fiber 165 composed of a single mode fiber or the like.

Although a Michelson-type interferometer is employed in this embodiment, it is possible to properly adopt an arbitrary type of interferometer such as the Mach-Zehnder-type.

The spectrometer 180 includes a collimator lens 181, a diffraction grating 182, an image forming lens 183, and a CCD 184.

The diffraction grating 182 may be a transmission-type diffraction grating that transmits light, or may be a reflection-type diffraction grating that reflects light. Moreover, it is also possible to. use another photodetecting device such as a CMOS device, instead of the CCD 184.

The interference light LC having entered the spectrometer 180 is collimated by the collimator lens 181, and divided into spectra by the diffraction grating 182 (spectral resolution). The divided interference lights LC are formed into an image on the image pick-up surface of the CCD 184 by the image forming lens 183. The CCD 184 detects the respective spectral components of the interference light LC and converts the components into electric charges. The CCD 184 accumulates these electric charges and generates a detection signal.

Furthermore, the CCD 184 transmits this detection signal to the arithmetic and control unit 200. The accumulation time and accumulation timing of the electric charges and the transmission timing of the detection signal are controlled by, for example, the arithmetic and control unit 200.

### [Configuration of Arithmetic and Control Unit]

Next, the configuration of the arithmetic and control unit 200 will be described. The arithmetic and control unit 200 analyzes the detection signal inputted from the CCD 184 of the OCT unit 150, and forms an OCT image of the fundus oculi Ef. A method of this analysis is the same as in the conventional Fourier-Domain-OCT technique.

Further, the arithmetic and control unit 200 forms a two-dimensional image showing the morphology of the surface of the fundus oculi Ef based on the video signals outputted from the imaging devices 10 and 12 of the retinal camera unit 1A.

Furthermore, the arithmetic and control unit 200 controls each part of the retinal camera unit 1A and the OCT unit 150.

To control the retinal camera unit 1A, the arithmetic and control unit 200 executes, for example: control of output of the illumination lights by the observation light source 101 and the imaging light source 103; control of insertion/retraction of the exciter filters 105, 106 and the barrier filters 122, 123 to/from the optical path; control of the operation of a display device such as the LCD 140; control of movement of the illumination diaphragm 110 (control of the diaphragm value); control of the diaphragm value of the imaging diaphragm 121; and control of movement of the magnifying lens 124 (control of the magnification). Furthermore, the arithmetic and control unit 200 executes control of the operation of the Galvano mirrors 141A and 141B.

On the other hand, to control the OCT unit 150, the arithmetic and control unit 200 executes, for example: control of output of the low-coherence light L0 by the low-coherence light source 160; control of movement of the reference mirror 174; control of the rotation operation of the density filter 173 (the operation of changing the reduction amount of the light amount of the reference light LR); and control of the accumulation timing and the timing of signal output by the CCD 184.

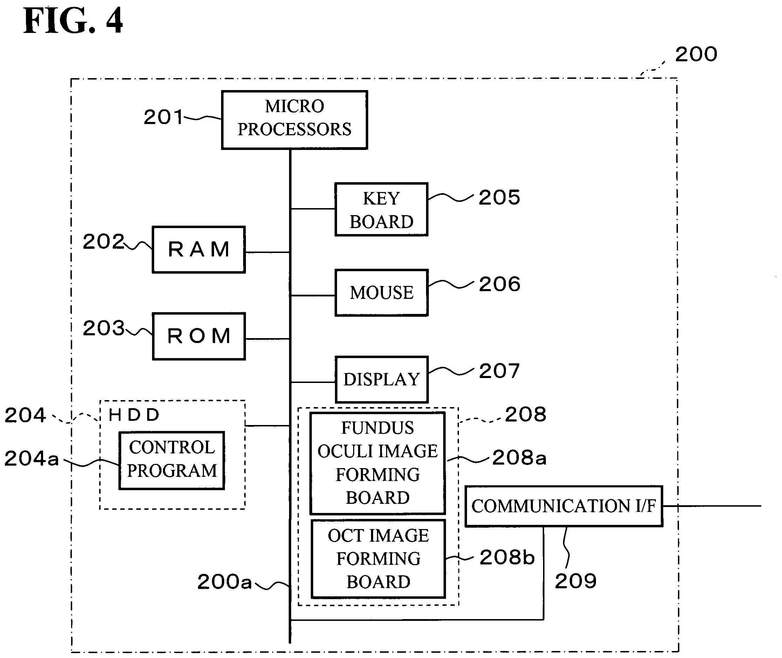

The hardware configuration of the arithmetic and control unit 200 will be described with reference to Fig. 4.

The arithmetic and control unit 200 is provided with a similar hardware configuration to that of a conventional computer. To be specific, the arithmetic and control unit 200 includes a microprocessor 201, a RAM 202, a ROM 203, a hard disk drive (HDD) 204, a keyboard 205, a mouse 206, a display 207, an image forming board 208, and a communication interface (I/F) 209. The respective parts are connected by a bus 200a.

The microprocessor 201 includes a CPU (Central Processing Unit), an MPU (Micro Processing unit) or the like. The microprocessor 201 reads out a control program 204a from the hard disk drive 204 and loads the program onto the RAM 202, thereby causing the fundus oculi observation device 1 to execute an operation characteristic to the present embodiment.

Further, the microprocessor 201 executes control of each of the aforementioned parts of the device, various arithmetic processes, and so on. Moreover, the microprocessor 201 receives a manipulation signal from the keyboard 205 or the mouse 206 and, in accordance with the content of the manipulation, controls each of the parts of the device. Furthermore, the microprocessor 201 executes control of a display process by the display 207, control of a process of transmission/reception of data and signals by the communication interface 209, and so on.

The keyboard 205, the mouse 206, and the display 207 are used as user interfaces of the fundus oculi observation device 1. For example, the keyboard 205 is used as a device for typing letters, figures or the like. The mouse 206 is used as a device for performing various kinds of manipulations for input into the display screen of the display 207.

Further, the display 207 is a display device such as an LCD or a CRT (Cathode Ray Tube) display, and displays various kinds of images such as an image of the fundus oculi Ef formed by the fundus oculi observation device 1, and also displays various kinds of screens such as a manipulation screen and a set-up screen.

The user interface of the fundus oculi observation device 1 is not limited to the above configuration, and may include, for example, a trackball, a joystick, a touch-panel LCD, and a control panel for ophthalmologic examination. As the user interface, it is possible to adopt an arbitrary configuration provided with a function of displaying/outputting information and a function of inputting information and manipulating the device.

The image forming board 208 is a dedicated electronic circuit that executes a process of forming (image data of) an image of the fundus oculi Ef. The image forming board 208 is provided with a fundus oculi image forming board 208a and an OCT image forming board 208b.

The fundus oculi image forming board 208a is a dedicated electronic circuit that forms image data of a fundus oculi image based on video signals from the imaging device 10 and the imaging device 12. The fundus oculi image forming board 208a functions as an example of the "imaging part" of the present invention, together with an optical system (the illumination optical system 100 and the imaging optical system 120) for capturing the fundus oculi image Ef'.

On the other hand, the OCT image forming board 208b is a dedicated electronic circuit that forms image data of a tomographic image of the fundus oculi Ef based on a detection signal from the CCD 184 of the OCT unit 150.

By installing the image forming board 208 described above, it is possible to increase the processing speed for the process of forming a fundus oculi image and a tomographic image.

The communication interface 209 transmits control signals from the microprocessor 201, to the retinal camera unit 1A or the OCT unit 150. Moreover, the communication interface 209 receives video signals from the imaging devices 10 and 12 and a detection signal from the CCD 184 of the OCT unit 150, and inputs the signals into the image forming board 208. In this process, the communication interface 209 inputs the video signals from the imaging devices 10 and 12, into the fundus oculi image forming board 208a, and inputs the detection signal from the CCD 184, into the OCT image forming board 208b.

Further, in a case that the arithmetic and control unit 200 is connected to a communication line such as a LAN (Local Area Network) or the Internet, it is possible to provide the communication interface 209 with a network adapter such as a LAN card or communication equipment such as a modem, thereby configuring to be capable of data communication via this communication network. In this case, it is possible to install a server that stores the control program 204a onto the communication network and configure the arithmetic and control unit 200 as a client terminal of the server, thereby causing the fundus oculi observation device 1 to operate.

### [Configuration of Control System]

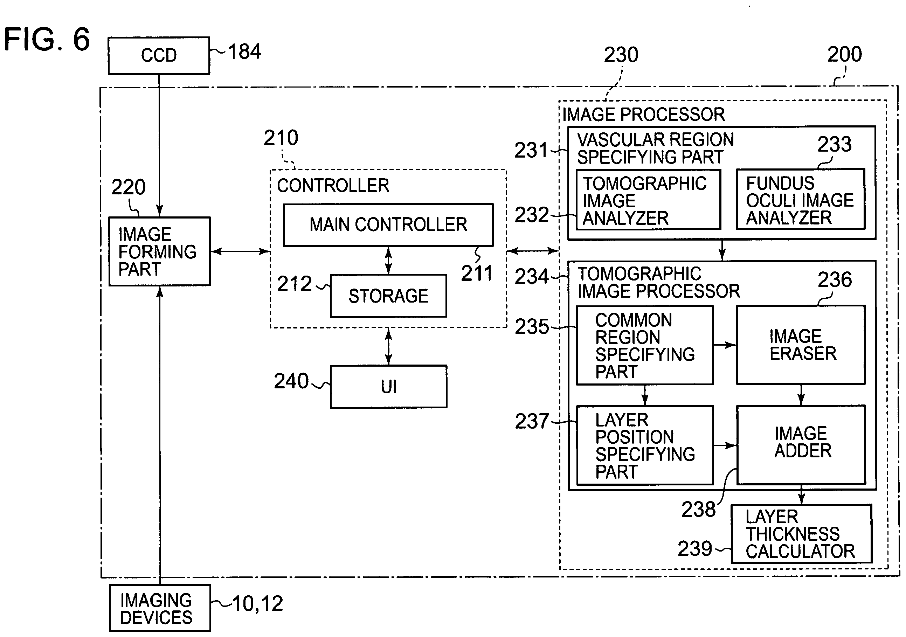

Next, the configuration of a control system of the fundus oculi observation device 1 will be described with reference to Figs. 5 and 6.

### (Controller)

The control system of the fundus oculi observation device 1 is configured mainly by a controller 210 of the arithmetic and control unit 200. The controller 210 includes the microprocessor 201, the RAM 202, the ROM 203, the hard disk drive 204 (the control program 204a), the communication interface 209, and so on.

The controller 210 is provided with a main controller 211 and a storage 212. The main controller 211 executes the aforementioned various controls. To be specific, the main controller 211 functions as an example of the "controller" of the present invention, and controls the display 240A to display a tomographic image of the fundus oculi Ef.

The storage 212 stores various kinds of data. The data stored in the storage 212 is, for example, the image data of an OCT image, the image data of the fundus oculi image Ef', subject information, and so on. The subject information is information on a subject, such as the ID and name of a patient. The main controller 211 executes a process of writing the data into the storage 212, and a process of reading out the data from the storage 212.

### (Image Forming Part)

An image forming part 220 forms the image data of the fundus oculi images Ef' based on the video signals from the imaging devices 10 and 12.

Further, the image forming part 220 forms the image data of a tomographic image of the fundus oculi Ef based on the detection signal from the CCD 184. This process includes, for example, noise elimination (noise reduction), filtering, FFT (Fast Fourier Transform), and so on. For example, the image forming part 220 determines the pixel value (the luminance value) based on the intensity of the detection signal, more specifically, the intensities of frequency components, thereby forming the image data of a tomographic image.

The image forming part 220 includes the image forming board 208, the communication interface 209, and so on. In this specification, "image data" may be identified with an "image" displayed based thereon.

### (Image Processor)

An image processor 230 executes various image processing and analysis processes on the image data of an image formed by the image forming part 220. For example, the image processor 230 executes various correction processes such as luminance correction and dispersion correction of an image.

Further, the image processor 230 executes an interpolation process of interpolating pixels between tomographic images formed by the image forming part 220, thereby forming the image data of a three-dimensional image of the fundus oculi Ef.

The image data of a three-dimensional image means such image data that the positions of the pixels are defined by the three-dimensional coordinates. An example of the image data of a three-dimensional image is image data composed of three-dimensionally arranged voxels. This image data is referred to as volume data, voxel data, or the like. For displaying an image based on the volume data, the image processor 230 executes a rendering process (such as volume rendering and MIP (Maximum Intensity Projection)) on this volume data, and forms the image data of a pseudo three-dimensional image taken from a specified view direction. On a display device such as the display 207, a pseudo three-dimensional image based on this image data is displayed.

Further, it is also possible to form stack data of a plurality of tomographic images as the image data of a three-dimensional image.

Stack data is image data obtained by three-dimensionally arranging a plurality of tomographic images obtained along a plurality of scan lines, based on the positional relation of the scan lines.

### (Vascular Region Specifying Part)

The image processor 230 is provided with a vascular region specifying part 231. The vascular region specifying part 231 is provided with a tomographic image analyzer 232 and a fundus oculi image analyzer 233.

The tomographic image analyzer 232 analyzes a tomographic image of the fundus oculi Ef and extracts a vascular region in this tomographic image. The tomographic image analyzer 232 is an example of the "first specifying part" of the present invention. On the other hand, the fundus oculi image analyzer 233 analyzes the fundus oculi image Ef' and extracts a vascular region in the fundus oculi image Ef'.

The fundus oculi image analyzer 233 is an example of the "second specifying part" of the present invention.

Here, the vascular region means an image region corresponding to a blood vessel of the fundus oculi Ef. Moreover, in a tomographic image, the vascular region may include, in addition to an image region corresponding to the cross section of a blood vessel, an image region located below the abovementioned image region (in the z-direction shown in Fig. 1). That is to say, the vascular region can be an image region corresponding to the position of a blood vessel when the fundus oculi is seen from the cornea side of the eye E. In other words, in a case that the coordinate values of a blood vessel in the xyz coordinate system are (x,y,z), the position of a vascular region can be expressed by coordinate values (x,y) obtained by projecting the coordinate values (x,y,z) to the xy plane.

### (Tomographic Image Analyzer)

An example of a process executed by the tomographic image analyzer 232 will be described. For this purpose, a tomographic image of the fundus oculi Ef will be described. In the fundus oculi Ef, layers such as the retina and the choroidea exist. Moreover, the retina has the internal limiting membrane, the nerve fiber layer, the ganglion cell layer, the inner plexiform layer, the inner nuclear layer, the outer plexiform layer, the outer nuclear layer, the external limiting membrane, the photoreceptor cell layer and the retinal pigment epithelium layer in order from the fundus oculi surface side in the depth direction. A tomographic image of the fundus oculi Ef describes the stratiform morphology of these layers.

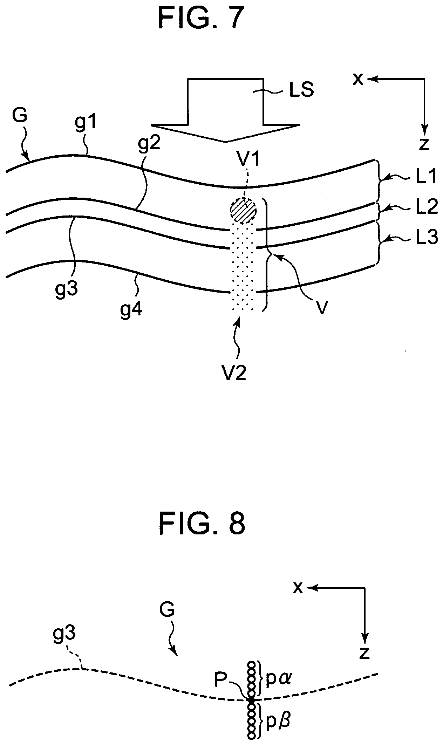

A tomographic image G shown in Fig. 7 depicts layer regions LI, L2 and L3 corresponding to the layers of the fundus oculi Ef and boundary regions g1, g2, g3 and g4 corresponding to the boundaries of the layers. Symbol VI in Fig. 7 denotes an image region corresponding to the cross section of the fundus blood vessel (a vascular cross-sectional region). Moreover, symbol V2 denotes an image region located just below the vascular cross-sectional region VI (a just-below-blood-vessel region). A vascular region V denotes an image region including the vascular cross-sectional region VI and the just-below-blood-vessel region V2. Symbol LS denotes a radiation direction of the signal light at the time of acquisition of the tomographic image G.

The vascular region V is not clearly displayed because of noise caused by a vascular wall, blood, blood flow or the like. Therefore, in the vascular region V, the layer regions L2 and L3 and the boundary regions g2-g4 are not clearly depicted.

In a first process example, the tomographic image analyzer 232 firstly analyzes a tomographic image and specifies a predetermined layer position. This predetermined layer position shall be, for example, the IS/OS position. Next, the tomographic image analyzer 232 extracts, from the tomographic image, a plurality of pixels located along the depth direction (+z direction and/or -z direction) of the fundus oculi Ef with respect to a pixel on the IS/OS position in the tomographic image.

A specific example of this process is shown in Fig. 8. The boundary region g3 shall be the IS/OS position. Symbol P denotes an arbitrary pixel on the boundary region g3. The tomographic image analyzer 232 extracts, from the tomographic image G, pixels pα (α=1-5) located on the side closer to the fundus oculi surface than the pixel P (-z direction) and pixels pβ (β=1-5) located just below the pixel P (+z direction).

The number of the extracted pixels is arbitrary. Moreover, the number of the extracted pixels may be identical or different in the +z direction and the -z direction. Moreover, only the pixels along the +z direction may be extracted, or only the pixels along the -z direction may be extracted. Moreover, in a case that there is no pixel on the boundary region g3, it is possible to regard a pixel at the closest position to the boundary region g3 as a pixel on the boundary region g3.

Next, the tomographic image analyzer 232 acquires the respective pixel values (the luminance values) of the pixels pα and pβ (and pixel P), and calculates a statistic representing variation of these pixel values. As this statistic, it is possible to use an arbitrary value that, when a plurality of pixel values are assumed as the population, defines variation of the plurality of pixel values, such as standard deviation or variance.

Next, the tomographic image analyzer 232 determines whether this statistic is included in a predetermined range. For example, in a case that the statistic is standard deviation or variance, it is possible to set a range equal to or less than a certain threshold as the predetermined range. To be specific, in a case that the threshold is denoted by Σ and the statistic corresponding to the pixel P is standard deviation σ(P), the tomographic image analyzer 232 determines whether σ(P) ≤ Σ is satisfied.

The threshold Σ is set based on the following characteristic of the tomographic image G, for example. The tomographic image G is an image showing the fine structure (the layer region and the boundary region) of the fundus oculi Ef, but cannot represent the fine structure in the vascular region. In a case that the tomographic image G is a luminance image, the vascular region is represented almost uniformly in black. That is to say, the pixels in the vascular region almost uniformly have low luminance values. The threshold Σ is used for determining whether the pixel on the boundary region g3 is a pixel in the vascular region or not. For example, this threshold Σ can be determined by, for a number of tomographic images, comparing the standard deviation of the luminance values of pixels in the vascular region with the standard deviation of the luminance values of pixels of the other image region and statistically processing (for example, averaging) the comparison result. The method for determining the threshold Σ is not limited to the above one. Moreover, statistics other than standard deviation can also be determined in the same way.

The tomographic image analyzer 232 executes such determination on each pixel P on the boundary region g3. Then, the tomographic image analyzer 232 specifies such a pixel that the statistic is included in the predetermined value. In the above specific example, the tomographic image analyzer 232 specifies such a pixel P on the boundary region g3 that the standard deviation σ(P) is equal to or less than the threshold Σ. Consequently, a set S of pixels shown below is obtained: S = {the pixel P on the boundary region g3: σ(P) ≤ Σ}.

The set S is a set of pixels determined to be located in the vascular region among the pixels P on the boundary region g3. The tomographic image analyzer 232 specifies the vascular region in the tomographic image in the above manner. This is the end of the description of the first example of the process.

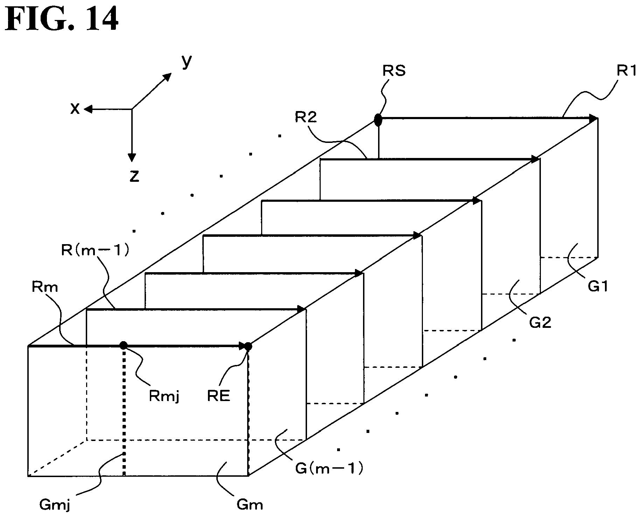

A second process example by the tomographic image analyzer 232 will be described. In a case that the second process example is applied, a plurality of tomographic images at different cross-sectional positions are acquired in advance. The plurality of tomographic images have cross sections parallel to each other, for example (refer to tomographic images G1-Gm shown in Fig. 14).

The tomographic image analyzer 232 firstly accumulates the plurality of tomographic images in the depth direction (the z-direction) of the fundus oculi Ef, respectively, to form an accumulated image.

This process is executed in the following manner, for example.

The tomographic image is an image formed by arranging depthwise images (one-dimensional images) at the target positions (the scan points) of the signal light LS. The tomographic image analyzer 232 accumulates the pixel values (luminance values) of pixels in the respective one-dimensional images, thereby forming an accumulated image.

The accumulated image is an image that artificially represents the surface morphology of the fundus oculi Ef in a scan region of the signal light LS, and is a similar image to the fundus oculi image Ef'.

After the description of Fig. 14, an example of a process of forming the accumulated image from the tomographic images G1-Gm will be described.

Next, the tomographic image analyzer 232 analyzes the accumulated image and obtains running position information that represents the running position of a blood vessel in the fundus oculi Ef. The accumulated image is an image that artificially represents the surface morphology of the fundus oculi Ef as described above. The accumulated image includes an image corresponding to the blood vessel of the fundus oculi Ef (a vascular region).

The tomographic image analyzer 232 extracts the vascular region in the accumulated image, for example, in the following manner.

Firstly, the tomographic image analyzer 232 executes a predetermined filtering process on the accumulated image. In this filtering process, for example, a process for making it easy to distinguish the vascular region in the accumulated image from other image regions is executed, such as a tone conversion process, an image enhancement process, a contrast conversion process, an edge detection process, an image averaging process and an image smoothing process.

Next, the tomographic image analyzer 232 binarizes the accumulated image based on a predetermined threshold. This threshold is set in advance based on, for example, the result of analysis of a number of accumulated images. It is also possible to, based on a histogram of the distribution of the pixel values (the luminance values) in an accumulated image, obtain a threshold unique to the accumulated image, and execute the binarizing process based on this threshold. By such a binarizing process, the vascular region in the accumulated image is enhanced.

The tomographic image analyzer 232 extracts the vascular region based on the pixel values (the luminance values) of the accumulated image after the binarizing process. Then, the tomographic image analyzer 232 specifies the position of the vascular region in the accumulated image, and regards the position information of this vascular region as the running position information. Considering a tomographic image is defined by the xyz coordinates and an accumulated image is formed based on tomographic images, the accumulated image is an image defined by the xyz coordinates (or the xy coordinates). Accordingly, the running position information is the position information of the vascular region in the accumulated image defined by the coordinate values of the xyz coordinate system (or the xy coordinate system).

Finally, the tomographic image analyzer 232 specifies the vascular region in the tomographic image based on the running position information. In this process, it is possible to specify the vascular region in the tomographic image at an arbitrary cross-sectional position of the fundus oculi Ef.

For example, since the coordinate system defining the tomographic image used for the process of forming the accumulated image is the same as the coordinate system defining the accumulated image, an image region in the tomographic image having the same coordinate values as the vascular region in the accumulated image is specified, and this image region is set as the vascular region.

Further, in the tomographic image that the cross section is set at an arbitrary position within a definition region of the accumulated image, it is possible to specify a vascular region in the following manner, for example. The tomographic image is formed based on image data of a three-dimensional image. Since the coordinate system defining the accumulated image and the coordinate system defining the image data of the three-dimensional image are the same, an image region in the tomographic image having the same coordinate values as the vascular region in the accumulated image is specified, and this image region is set as the vascular region.

Also in a tomographic image acquired by scanning the definition region of the accumulated image with the signal light LS not based on the image data of the three-dimensional image, it is possible to specify the vascular region in a similar way by referring to scan position information descried later. This is the end of the description of the second process example.

A third process example by the tomographic image analyzer 232 will be described. In a case that the third process example is applied, a plurality of tomographic images as in the second process example and the fundus oculi image Ef' are acquired in advance. In the third process example, a vascular region in the fundus oculi image Ef' shall be specified by the fundus oculi image analyzer 233 in advance (described later).

Based on the vascular region of the fundus oculi Ef', the tomographic image analyzer 232 obtains running position information that represents the running position of a blood vessel in the fundus oculi Ef.

Next, the tomographic image analyzer 232 forms an accumulated image as in the second process example. The accumulated image is, as mentioned before, an image that artificially represents the surface morphology of the fundus oculi Ef, and an image identical to the fundus oculi image Ef'.

Next, the tomographic image analyzer 232 executes position matching of the fundus oculi image Ef' and the accumulated image.

This process can be executed by, for example, executing position matching of a characteristic region in the fundus oculi Ef' (a character region) and a characteristic region in the accumulated image.

The character region is, for example, a vascular region, an image region corresponding to the optic papilla, an image region corresponding to the macula, a branch position of blood vessels, and so on. The position matching of images can be executed by, for example, using known image processing such as pattern matching or image correlation. Through this position matching process, a coordinate transformation equation between the coordinate system defining the fundus oculi image Ef' and the coordinate system defining the accumulated image is obtained.

Next, the tomographic image analyzer 232 specifies an image region in the accumulated image corresponding to the vascular region in the fundus oculi image Ef', based on the result of the position matching described above. For example, this process is executed by using the above coordinate transformation equation to transform the coordinate values of the vascular region in the fundus oculi image Ef' shown in the running position information into coordinate values of the coordinate system defining the accumulated image. Consequently, the image region (the vascular region) in the accumulated image corresponding to the vascular region in the fundus oculi image Ef' is specified.

Next, the tomographic image analyzer 232 specifies a crossing region of the vascular region in the accumulated image and the cross section of the tomographic image. This process can be executed in the same manner as in the second process example. This crossing region is defined in an image region corresponding to the fundus oculi surface.

Finally, the tomographic image analyzer 232 specifies the vascular region in the tomographic image so as to include this crossing region. The crossing region is defined in the image region corresponding to the fundus oculi surface as described above. The tomographic image analyzer 232 sets an image region just below the crossing region in the tomographic image as the vascular region. For example, in a case that the coordinate values of the crossing region are (x,y), the tomographic image analyzer 232 sets an image region defined by coordinate values (x,y,z) as the vascular region.

Thus, in the third process example, the vascular region in the fundus oculi image Ef' is specified, the image region in the accumulated image corresponding to this vascular region is specified, and the common region to this image region and the tomographic image is set as the vascular region in the tomographic image. In general, the fundus oculi image Ef' is a clearer image than an accumulated image.

Therefore, a vascular region extracted from the fundus oculi image Ef' is higher in accuracy and precision than a vascular region extracted from an accumulated image (the second process example).

Accordingly, in the third example of the process, it is possible to set a vascular region with higher accuracy and precision than in the second process example. Since the accuracy and precision in the third process example depends on the position matching process between the fundus oculi image Ef' and the accumulated image, it is necessary to favorably execute this position matching process.

### (Fundus Oculi Image Analyzer)

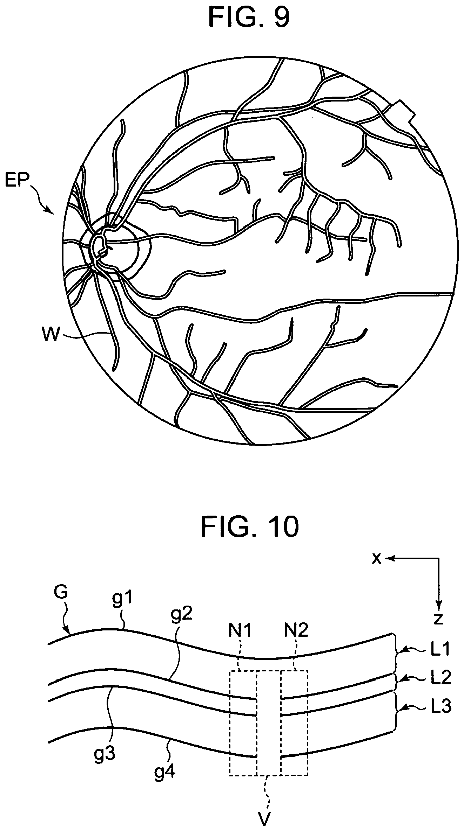

An example of a process executed by the fundus oculi image analyzer 233 will be described. For this description, the fundus oculi image Ef' will be described. In the fundus oculi image Ef', as shown in Fig. 9, an image region (a vascular region) W corresponding to a blood vessel located on (or near) the surface of the fundus oculi Ef exists.

The vascular region W is significantly clearly depicted by executing fluorography, for example.

The fundus oculi image analyzer 233, for example, executes a filtering process as in the second example on the fundus oculi image Ef', and detects a change in pixel value (luminance value) in the x-direction and y-direction to specify a vascular region in the fundus oculi image Ef'.

Further, it is also possible to specify the vascular region by executing threshold processing for distinguishing the vascular region from the other image region on the fundus oculi image Ef'. This threshold may be set in advance, or may be set for each fundus oculi image Ef'. For example, the threshold in the former case can be statistically obtained by analyzing a number of fundus oculi images having been clinically acquired. Moreover, it is possible to analyze fundus oculi images of the eye E having been captured in the past and acquire a threshold for each of the eyes E. On the other hand, the threshold in the latter case can be set by, for example, generating a histogram of the pixel values of pixels in the fundus oculi image Ef' and referring to this histogram.

### (Tomographic Image Processor)

The image processor 230 is provided with a tomographic image processor 234. The tomographic image processor 234 executes predetermined image processing on the tomographic image based on the vascular region specified by the vascular region specifying part 231. The tomographic image processor 234 is an example of the "image processor" of the present invention. The tomographic image processor 234 is provided with a common region specifying part 235, an image eraser 236, a layer position specifying part 237 and an image adder 238.

### (Common Region Specifying Part)

The common region specifying part 235 specifies, of the vascular region in the tomographic image, a region common to the vascular region in the fundus oculi image Ef'.

An example of a process executed by the common region specifying part 235 will be described. The common region specifying part 235 accepts, from the vascular region specifying part 231, positional information of the vascular region in the tomographic image and the positional information of the vascular region in the fundus oculi image Ef'. The former positional information includes coordinate values of the vascular region in a coordinate system (for example, the xyz coordinate system) defining the tomographic image. Moreover, the latter positional information includes coordinate values of the vascular region in a coordinate system (for example, the xy coordinate system) defining the fundus oculi image Ef'.

The common region specifying part 235 executes position matching of the tomographic image and the fundus oculi image Ef' as needed. The common region specifying part 235 can execute this position matching process, for example, with the accumulated image as the tomographic image analyzer 232 does.

Next, the common region specifying part 235 compares the positional information of the vascular region in the tomographic image with the positional information of the vascular region in the fundus oculi image Ef', and specifies a vascular region included in both the images. This process is executed by, for example, comparing a set of the coordinate values of the vascular region in the tomographic image with a set of the coordinate values of the vascular region in the fundus oculi image Ef' and specifying the coordinate values belonging to both the sets. Thus, a vascular region (a common region) common to the tomographic image and the fundus oculi image Ef' is specified.

The common region specifying part 235 specifies an image region in the tomographic image corresponding to this common region.

That is to say, the common region specifying part 235 specifies, of the vascular region in the tomographic image, a region common to the vascular region in the fundus oculi image Ef'.

### (Image Eraser)

The image eraser 236 erases the image region (the common region) specified by the common region specifying part 235, from the tomographic image. This process can be executed by, for example, changing the pixel value of each of the pixels within the common region into a predetermined pixel value. As a specific example thereof, in a case that the tomographic image is a luminance image, the luminance value of each of the pixels within the common region is set to zero.

Although it is sufficient that the region erased by the image eraser 236 includes at least part of the common region, it is desirable that the erased region is an image region of the whole common region or an image region including the common region.

### (Layer Position Specifying Part)

The layer position specifying part 237 specifies the position of the layer in the tomographic image. For this purpose, firstly, the layer position specifying part 237 executes preprocessing for making it easy to obtain the layer position of the tomographic image as needed. As this preprocessing, for example, image processing such as tone conversion, image enhancement, threshold processing, contrast conversion, binarizing, edge detection, image averaging, image smoothing or filtering is executed. These image processing can also be properly executed in combination.

Next, the layer position specifying part 237 analyzes the pixel values (for example, the luminance values) of the pixels composing the tomographic image for each line along the depth direction of the fundus oculi Ef. There is no need to execute this analysis process on the common region specified by the common region specifying part 235.

The tomographic image is composed of a plurality of depthwise images arranged along a predetermined cross section (refer to an image Gij shown in Fig. 14). The layer position specifying part 237 sequentially refers to the pixel values of the pixels composing the depthwise image along the depth direction, thereby specifying a pixel located on the boundary between the adjacent layers. This process can be executed by using, for example, a filter that extends only in the depth direction (for example, a line filter such as a differential filter) or a filter that extends in the depth direction and a direction orthogonal thereto (an area filter). Such a filter is prestored in a hard disk drive 204, for example.

Thus, the layer position specifying part 237 obtains an image region corresponding to the boundary position between layers, and also obtains an image region corresponding to a layer. Since the fundus oculi Ef is composed such that a plurality of layers are stacked, specification of a layer is synonymous with specification of the boundary between layers.

As mentioned before, the fundus oculi Ef has a plurality of layers. The layer position specifying part 237 specifies at least one layer position (or boundary position between layers) from among these layers.

To be specific, the layer position specifying part 237 specifies the IS/OS position (the boundary position between the inner nuclear layer and the outer plexiform layer). It is possible to, for example, extract the inner nuclear layer and the outer plexiform layer, respectively, and specify the boundary position between these layers as the IS/OS position. Moreover, it is also possible to specify the IS/OS position by change in luminance value of the tomographic image.

Moreover, it is also possible to specify the IS/OS position by referring to a distance from a reference position (the fundus oculi surface, the retinal pigment epithelial layer, or the like) in the tomographic image.

The "layer" shall include the abovementioned respective layers composing the retina, and also the choroidea, the sclera and external tissues thereof. Moreover, the boundary position between the layers shall include the boundary position between the abovementioned layers composing the retina, and also the boundary position between the internal limiting membrane and the vitreous body, the boundary position between the retinal pigment epithelial layer and the choroidea, the boundary position between the choroidea and the sclera, the boundary position between the sclera and external tissues thereof, and so on.

When the layer position in the image region excluding the vascular region in the tomographic image is specified by the above process, the layer position specifying part 237 estimates the layer position in the region (the common region) erased by the image eraser 236, based on the layer position in the neighborhood region of the common region. In this process, for example, the boundary region between the layers is specified based on the pixel values of the pixels within the neighborhood region of the common region, and the boundary position between the layers in the common region is estimated based on the morphology of the boundary region of the layers. An example of this estimation process will be described below.

The layer position specifying part 237 firstly sets a neighborhood region of each of the common regions in the tomographic images. It is desirable that the neighborhood regions are set on both the sides of the common region in order to increase the precision of the estimation. As a specific example, in a case that the vascular region V of the tomographic image G (an image in the xz cross section) of Fig. 7 is a common region, an image region N1 adjacent to the vascular region V on the +x side and an image region N2 adjacent to the vascular region V on the -x side are set as the neighborhood regions.

Here, the width of the neighborhood region (the distance in the x-direction in the above example) can be set in advance (for example, about ten pixels to tens of pixels). Moreover, for example, it is possible to set, for each tomographic image, a neighborhood region having such a width that allows precise grasp of the morphology of the boundary between the layers.

Next, the layer position specifying part 237 obtains the positions of the boundary regions between the layers at the boundary between the respective neighborhood regions on both the sides of the common region and the common region. Subsequently, based on the obtained positions, the layer position specifying part 237 obtains a straight line connecting the boundary regions on both the sides. Then, the positions on this straight line shall be the boundary region of the layers in the common region.

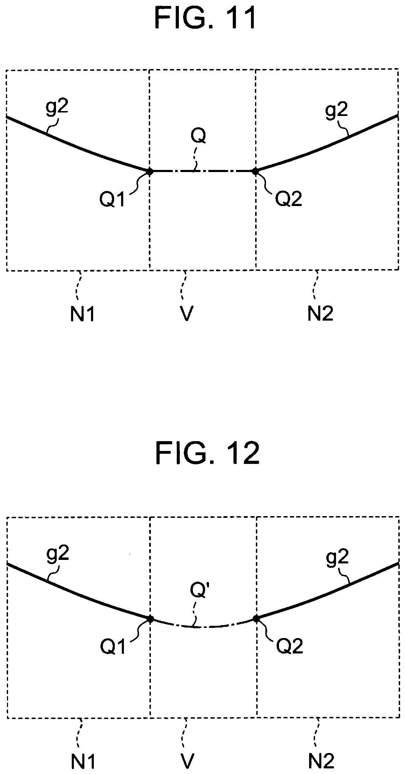

As a specific example of this process, a process of estimating a corresponding site to the boundary region g2 in the vascular region V (the common region) in Fig. 10 will be described. Firstly, the layer position specifying part 237 executes a smoothing process on the boundary region g2 in each of the neighborhood regions N1 and N2 and converts the boundary region g2 into a curved line as needed. Next, the layer position specifying part 237 acquires positions Q1 and Q2 of the boundary region g2 at the boundary between the respective neighborhood regions N1, N2 and the vascular region V (the boundaries on both the sides of the vascular region V) (refer to Fig. 11). Then, the layer position specifying part 237 obtains a straight line Q connecting the positions Q1 and Q2. The straight line Q can be easily calculated from the coordinate values of the positions Q1 and Q2. A position on the straight line Q becomes an estimated position of the boundary region g2 in the vascular region V.

Alternatively, it is possible to estimate the position of the boundary region g2 in the vascular region V by using a curved line instead of a straight line. As a specific example thereof, the layer position specifying part 237 obtains the positions Q1 and Q2 in the same manner as described above, and also obtains the slope of the boundary region g2 at each of the positions Q1 and Q2. The value of the slope can be obtained from the slope at the respective points of the boundary region g2 within the neighborhood regions N1 and N2. Then, the layer position specifying part 237 obtains a spline curve Q' connecting the positions Q1 and Q2 based on the positions Q1, Q2 and the slope (refer to Fig. 12). A position on the spline curve Q' becomes an estimated position of the boundary region g2 in the vascular region V.

In the above example, the common region exists at a position other than the end part of the tomographic image. In a case that the common region exists at the end part of the tomographic image, it is impossible to consider the neighborhood regions on both the sides.

Therefore, it is possible to consider only the neighborhood region on one of the sides and process in the same manner as described above. Moreover, even when the common region exists at a position other than the end part, it is possible to consider only the neighborhood region on one of the sides for the purpose of shortening the process time.

Further, the process may be changed in accordance with the width of the common region (the distance between the positions Q1 and Q2). For example, it is possible to shorten the process time by the estimation process using a straight line when the width is a predetermined distance or less, whereas it is possible to increase the precision and accuracy by the estimation process using a curved line when the width exceeds the predetermined distance.

### (Image Adder)

The image adder 238 adds an image representing the layer position specified by the layer position specifying part 237 to the image region erased by the image eraser 236. Consequently, for example, as shown in Figs. 11 and 12, an image of the straight line Q and an image of the spline curve Q' that represent the layer position (the boundary position between the layers) are added into the common region (the vascular region V).

In this embodiment, the common region is once erased from the tomographic image, and then, an image of the layer position is added to the common region, but the process is not limited thereto. For example, it is possible to process so as to replace an original image within the common region in the tomographic image with an image of the layer position, which is substantially the same process as described above.

### (Layer Thickness Calculator)

The layer thickness calculator 239 calculates the layer thickness of a predetermined site of the fundus oculi Ef based on the tomographic image. To be specific, the layer thickness calculator 239 obtains the layer thickness of a predetermined site of the fundus oculi Ef in the common region (the vascular region) based on the image added by the image adder 238. The layer thickness calculator 239 is an example of the "calculator" of the present invention.

Here, the predetermined site of the fundus oculi Ef means one or more layers of the plurality of layers in the fundus oculi Ef mentioned above. For example, the retinal pigment epithelial layer alone is equivalent to the "predetermined site," and a plurality of layers from the internal limiting membrane to the inner nuclear layer are also equivalent to the "predetermined site."

Further, the thickness of the "predetermined site" to be calculated is, for example, the thickness from the internal limiting membrane to the nerve fiber layer (a nerve fiber layer thickness), the thickness from the internal limiting membrane to the inner nuclear layer (the IS/OS position of photoreceptor cells) (a retina thickness), the thickness from the internal limiting membrane to the retinal pigment epithelial layer (a retina thickness), and so on. Among these three examples, the second and third examples are defined differently, but both represent the retina thickness.

An example of a process executed by the layer thickness calculator 239 will be described. As mentioned above, the layer position specifying part 237 specifies the positions (the boundary positions) of the layers of the fundus oculi Ef in the tomographic image. In this process, at least two boundary positions (that is, at least one layer) are specified. The layer thickness calculator 239 calculates the distance between predetermined two boundary positions among the specified boundary positions.

To be specific, the layer thickness calculator 239 calculates the distance (the depthwise distance) between pixels corresponding to the two boundary positions, for the respective depthwise images composing the tomographic image. In this process, to each pixel of the depthwise image, coordinate values of the aforementioned xyz coordinate system are given (the x-coordinate value and y-coordinate value are constant, respectively). The layer thickness calculator 239 can calculate the distance between the pixels from these coordinate values. Moreover, the layer thickness calculator 239 can also calculate a target distance based on the number of pixels between the pixels corresponding to the two boundary positions and based on the distance (known) between adjacent pixels. The layer thickness in the common region can also be obtained in the same manner.

The layer thickness calculator 239 obtains the thickness of the layer at a plurality of positions of the fundus oculi Ef, and generates information (layer thickness distribution information) representing the distribution of the thicknesses of the layer. The layer thickness distribution information is, for example, a layer thickness graph that graphs the distribution of the thicknesses of the layer in a predetermined cross-sectional position. Moreover, a layer thickness distribution image that expresses one-dimensional or two-dimensional distribution of the thicknesses of the layer in colors corresponding to the thicknesses of the layer may be applied as the layer thickness distribution information.

The process of generating the layer thickness distribution information will be described more specifically. Information acquired by the process of calculating the layer thickness described above is information that relates the analysis position of the layer thickness to the value of the layer thickness. That is to say, as described above, the layer thickness is obtained for each depthwise image, and coordinate values of the xyz coordinate system (or the xy coordinate system) are given to each depthwise image. Thus, the layer thickness calculator 239 can relate the analysis position defined by the xyz coordinate system (or the xy coordinate system) to the value of the layer thickness calculated from the depthwise image at the analysis position.

The layer thickness calculator 239 can generate the layer thickness distribution information by aligning the information relating the analysis position to the value of the layer thickness in accordance with, for example, the analysis position.

Further, the layer thickness calculator 239 can generate the layer thickness graph by selecting information included in a predetermined cross-sectional position (the position is defined by the xyz coordinate system or the xy coordinate system) from information of the layer thickness at a plurality of positions, and aligning the values of the layer thicknesses of the selected information in accordance with the analysis positions. For example, by defining the analysis positions on the horizontal axis and plotting the values of the layer thicknesses on the vertical axis based on the thus generated information, it is possible to display this layer thickness graph. This display process is executed by the main controller 211.

Further, the layer thickness calculator 239 can generate a layer thickness distribution image (image data) by selecting information included in a predetermined region (the position is defined by the xyz coordinate system or the xy coordinate system) from information of the layer thickness at a plurality of positions, aligning the values of the layer thicknesses of the selected information in accordance with the analysis positions, and giving colors corresponding to the values of the layer positions. By displaying each pixel within the predetermined region in given color based on this image data, it is possible to display the layer thickness distribution image. This display process is executed by the main controller 211.

The image processor 230 described above includes the microprocessor 201, the RAM 202, the ROM 203, the hard disk drive 204 (the control program 204a) and so on.

### (User Interface)

A user interface (UI) 240 is provided with a display 240A and a manipulation part 240B. The display 240A is composed of a display device such as the display 207. The display 240A is an example of the "display" of the present invention. Moreover, the manipulation part 240B is composed of an input device and a manipulation device such as the keyboard 205 and the mouse 206

### [Scan with Signal Light and Image Processing]

An example of the pattern of scan with the signal light LS and the pattern of image processing will be described. Scan with the signal light LS is executed by the scan unit 141. To be specific, scan with the signal light LS is executed by control of the mirror drive mechanisms 241 and 242 by the controller 210 to change the directions of the reflecting surfaces of the Galvano mirrors 141A and 141B.

The Galvano mirror 141A scans with the signal light LS in the horizontal direction (the x-direction in Fig. 1). The Galvano mirror 141B scans with the signal light LS in the vertical direction (the y-direction in Fig. 1). Moreover, by operating both the Galvano mirrors 141A and 141B simultaneously, it is possible to scan with the signal light LS in any direction on the xy plane.

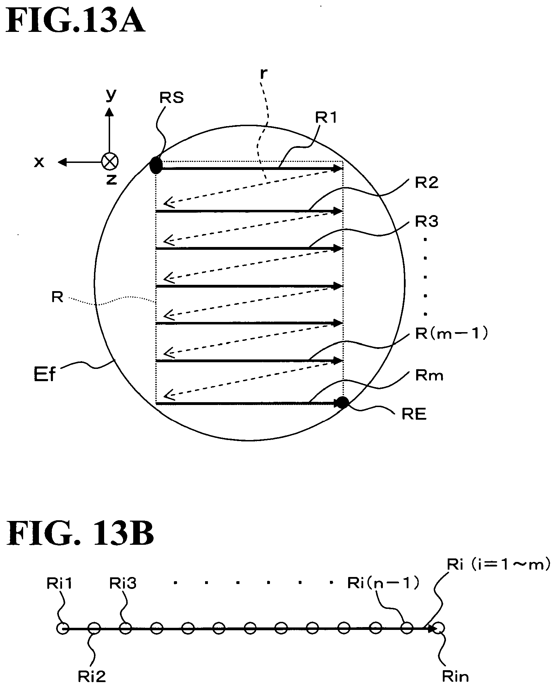

Figs. 13A and 13B show an example of the pattern of scan with the signal light LS for forming an image of the fundus oculi Ef. Fig. 13A shows an example of the pattern of scan with the signal light LS, when the fundus oculi Ef is seen from a direction where the signal light LS enters the eye E (that is, seen from -z side toward +z side in Fig. 1). Moreover, Fig. 13B shows an example of the pattern of arrangement of scan points (measurement positions) on each scan line on the fundus oculi Ef.

As shown in Fig. 13A, scan with the signal light LS is executed within a rectangular scan region R. Within this scan region R, a plurality of (m lines of) scan lines R1-Rm along the x-direction are set.

Scan lines Ri (i=1-m) are arranged in the y-direction. The direction of each of the scan lines Ri (the x-direction) will be referred to as the "main scan direction" and the direction orthogonal thereto (the y-direction) will be referred to as the "sub-scan direction."

On each of the scan lines Ri, as shown in Fig. 13B, a plurality of (n pieces of) scan points Ril-Rin are set. The positions of the scan region R, scan lines Ri and scan points Rij are properly set before execution of a measurement.

In order to execute the scan shown in Figs. 13A and 13B, the controller 210 firstly controls the Galvano mirrors 141A and 141B to set the incident target of the signal light LS into the fundus oculi Ef to a scan start position RS (a scan point R11) on the first scan line R1.

Subsequently, the controller 210 controls the low-coherence light source 160 to flash the low-coherence light L0, thereby making the signal light LS enter the scan start position RS. The CCD 184 receives the interference light LC based on the reflected light of this signal light LS at the scan start position RS, accumulates electric charges, and generates a detection signal.

Next, the controller 210 controls the Galvano mirror 141A to scan with the signal light LS in the main scan direction to set the incident target to a scan point R12, and flashes the low-coherence light L0 to make the signal light LS enter the scan point R12. The CCD 184 receives the interference light LC based on the reflected light of this signal light LS at the scan point R12, accumulates electric charges, and generates a detection signal.

Likewise, the controller 210 controls to generate a detection signal corresponding to each of the scan points, by flashing the low-coherence light L0 at each of the scan points while sequentially moving the incident target of the signal light LS from a scan point R13 to R14, ----, R1(n-1), and R1n.

When measurement at a last scan point R1n on the first scan line R1 is finished, the controller 210 simultaneously controls the Galvano mirrors 141A and 141B to move the incident target of the signal light LS to a first scan point R21 on a second scan line R2, along a line switching scan r. Then, the controller 210 controls to execute the same measurement on each of scan points R2j (j=1-n) on this second scan line R2 and to generate detection signals corresponding to the respective scan points R2j.

Likewise, the controller 210 controls to execute a measurement on each of a third scan line R3, ----, an m-1th scan line R(m-1), an mth scan line Rm and to generate a detection signal corresponding to each scan point. Symbol RE on the scan line Rm denotes a scan end position corresponding to a scan point Rmn.

Thus, the controller 210 controls to generate m×n pieces of detection signals corresponding to m×n pieces of scan points Rij (i=1-m, j=1-n) within the scan region R. A detection signal corresponding to each of the scan points Rij may be denoted by Dij.

In the above control, when operating the Galvano mirrors 141A and 141 B, the controller 210 acquires position information (coordinates in the xy coordinate system) of each of the scan points Rij. This position information (scan position information) is referred to when an OCT image is formed, for example.

Next, an example of image processing when the scan shown in Fig. 13A and Fig. 13B is executed will be described.

The image forming part 220 forms tomographic images of the fundus oculi Ef along the respective lines Ri (the main scan direction).

Moreover, the image processor 230 forms a three-dimensional image of the fundus oculi Ef based on the tomographic images formed by the image forming part 220.

The tomographic image formation process includes a two-step arithmetic process as conventional. In the first step, based on each detection signal Dij, an image in the depth direction (the z-direction in Fig. 1) of the fundus oculi Ef at the scan point Rij is formed.

In the second step, the depthwise images at the scan points Ril-Rin are arranged based on the scan position information, and a tomographic image Gi along the scan line Ri is formed. Through the above process, m pieces of tomographic images G1-Gm are obtained.

The image processor 230 arranges the tomographic images G1-Gm based on the scan position information and executes an interpolating process of interpolating an image between the adjacent tomographic images Gi and G(i+1), thereby generating a three-dimensional image of the fundus oculi Ef. This three-dimensional image is defined by the three-dimensional coordinates (x,y,z) based on the scan position information, for example.

Further, the image processor 230 is capable of forming a tomographic image in an arbitrary cross-section, based on this three-dimensional image. When the cross-section is designated, the image processor 230 specifies the position of each scan point (and/or an interpolated depthwise image) on the designated cross-section, extracts a depthwise image (and/or an interpolated depthwise image) at each specified position from the three-dimensional image, and arranges a plurality of extracted depthwise images based on the scan position information or the like, thereby forming a tomographic image in the designated cross-section.

An image Gmj shown in Fig. 14 represents a depthwise image at the scan point Rmj on the scan line Rm. Likewise, a depthwise image at the scan point Rij formed in the aforementioned first-step is represented as an "image Gij."

Here, an example of a process of forming an accumulated image based on the tomographic images G1-Gm will be described. The tomographic image analyzer 232 accumulates the images Gij composing the tomographic image Gi in the depth direction (the z-direction) to form a dotted image.

"Accumulation in the depth direction" means a calculation of summing (projecting) the luminance values of pixels composing the image Gij in the depth direction. Therefore, the dotted image obtained by accumulating the image Gij has such a luminance value that the luminance values at the respective z-positions of the image Gij are summed in the depth direction. Moreover, the position of the dotted image has the same coordinate values as that of the image Gij in the xy-coordinate system.

The tomographic image analyzer 232 executes the abovementioned accumulation process on each of the m pieces of tomographic images G1-Gm obtained by a series of scans with the signal light LS. Consequently, an accumulated image formed by m × n pieces of dotted images that are two-dimensionally distributed in the scan region R is formed. This accumulated image is an image that represents the morphology of the surface of the fundus oculi Ef, as well as the fundus oculi image Ef' in the scan region R.

The scan pattern of the signal light LS by the fundus oculi observation device 1 is not limited to the abovementioned one. For example, it is possible to scan with the signal light LS only in the horizontal direction (the x-direction), only in the vertical direction (the y-direction), in the longitudinal and lateral directions like a cruciform, radially, circularly, concentrically, or helically. That is to say, as mentioned before, the scan unit 141 is configured to be capable of independently scanning with the signal light LS in the x-direction and the y-direction, so that it is possible to scan with the signal light LS along an arbitrary trajectory on the xy-plane.

### [USAGE PATTERN]

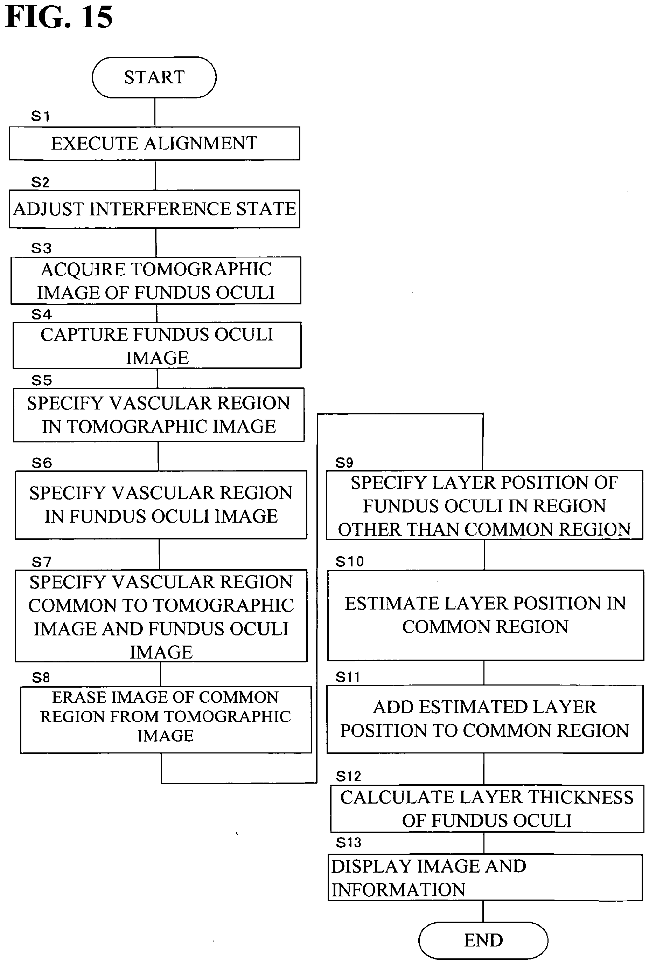

A usage pattern of the fundus oculi observation device 1 will be described. The flow chart shown in Fig.15 shows an example of the usage pattern of the fundus oculi observation device 1.

Firstly, alignment of an optical system with the eye E is executed (S1). The alignment is executed as in a conventional retinal camera. For example, the alignment is executed by adjusting the position of the retinal camera unit 1A while projecting an alignment bright point (not shown) to the eye E to observe the state thereof.

Next, the position of the reference mirror 174 is adjusted, and the interference state of the signal light and the reference light is adjusted (S2). This adjustment is executed so that an image at a desired depth position of the fundus oculi Ef becomes clear. The position adjustment of the reference mirror 174 may be manually performed by using the manipulation part 240B, or may be automatically performed.

Subsequently, in response to the predetermined manipulation, the main controller 211 controls the LCD 140 to project a fixation target to the eye E, and also controls the low-coherence light source 160, the scan unit 141, the CCD 184, the image forming part 220 and so on to acquire a tomographic image of the fundus oculi Ef (S3). The main controller 211 stores the acquired tomographic image into the storage 212.

Further, the main controller 211 controls the observation light source 101 (or the imaging light source 103), the imaging device 12 (the imaging device 10), the image forming part 220 and so on to capture a two-dimensional image of the surface of the fundus oculi Ef, namely, the fundus oculi image Ef' (S4). This process may be automatically started in response to completion of step S3, or may be started in response to a predetermined manipulation. Moreover, the fundus oculi image Ef' may be captured before the tomographic image is acquired. The main controller 211 stores the fundus oculi image Ef' into the storage 212.

Next, the tomographic image analyzer 232 specifies a vascular region in the tomographic image of the fundus oculi Ef (S5). Moreover, the fundus oculi image analyzer 233 specifies a vascular region in the fundus oculi image Ef' (S6). Step S5 and step S6 may be reversed, or both the processes may be executed in parallel.

Next, the common region specifying part 235 specifies, of the vascular region in the tomographic image, a vascular region (a common region) common to the vascular region in the fundus oculi image Ef' (S7).

Next, the image eraser 236 erases the image of the region specified as the common region from the tomographic image (S8).

Next, the layer position specifying part 237 specifies, based on the tomographic image, the layer position of the fundus oculi Ef in a region other than the region where the image has been erased (S9).

Furthermore, the layer position specifying part 237 estimates, based on the specified layer position, the layer position in the region (the common region) where the image has been erased (S10).

Next, the image adder 238 adds an image representing the layer position estimated in step S10 to the region (the common region) where the image has been erased at step S8 (S11).

Next, the layer thickness calculator 239 calculates the layer thickness of the fundus oculi Ef based on the tomographic image to which the image has been added at step S11 (S12). The layer thickness calculator 239 properly generates the aforementioned layer thickness graph or layer thickness distribution image.

The main controller 211 controls the display 240A to display various images or information having been processed above (S13). The information that can be displayed is, for example, the tomographic image acquired at step S3, the fundus oculi image Ef' captured at step S4, the image in which the vascular region specified at step S5 or step S6 is enhanced, the tomographic image in which the common region specified at step S7 is enhanced, the tomographic image from which the common region is erased at step S8, the tomographic image in which the layer position specified at step S9 is enhanced, the tomographic image in which the image added at step S11 is enhanced, the layer thickness graph or layer thickness distribution image obtained at step S12, or the like.

In particular, in the case of displaying a tomographic image of the fundus oculi Ef, the main controller 211 controls to display the tomographic image so that a region corresponding to the common region can be visually recognized. For example, it is possible to display a frame-like image surrounding the region corresponding to the common region, or change the display pattern (display color, contrast, or the like) of the image within the region.

Further, in the case of displaying a tomographic image from which the image of the region corresponding to the common region has been erased (or a tomographic image obtained by processing the above tomographic image), it is possible to visually recognize the region in the image, and therefore, the tomographic image may be displayed as it is.

### [ACTIONS AND EFFECTS]

The actions and effects of the fundus oculi observation device 1 as described above will be described.

The fundus oculi observation device 1 is provided with a function of forming a tomographic image of the fundus oculi Ef and a function of capturing the fundus oculi image Ef'. Furthermore, the fundus oculi observation device 1 acts to specify a vascular region in the tomographic image and a vascular region in the fundus oculi image Ef', respectively, obtain a common region of these vascular regions, and specify a region in the tomographic region corresponding to this common region.

According to the fundus oculi observation device 1, it is possible to specify, of the vascular region in the tomographic image, a region common to the vascular region of the fundus oculi image Ef'.

Therefore, it is possible to specify the vascular region in the tomographic image with higher accuracy than before based on both the images.

Further, in the case of forming a three-dimensional image from a plurality of tomographic images, by executing the process on the respective tomographic images, it is possible to specify a vascular region in the three-dimensional image with higher accuracy.

Further, since it is possible to display a tomographic image so that a region corresponding to the common region can be visually recognized, it is possible to present the position of the vascular region in the tomographic image with high accuracy.

Further, according to the fundus oculi observation device 1, it is possible to obtain the layer position in the vascular region common to that of the fundus oculi image Ef' based on the layer position of the neighborhood thereof. Therefore, it is possible to obtain the layer position of the vascular region with higher accuracy. Furthermore, the device acts to obtain the layer thickness in the vascular region based on the thus obtained layer position, it is possible to obtain the layer thickness in the vascular region with higher accuracy.

### [MODIFICATION]

The configuration described above is merely an example for favorably implementing the fundus oculi observation device relating to the present invention. Therefore, it is possible to properly apply an arbitrary modification within the scope of the present invention.

### [Modification 1 - not under the scope of the present invention]

Although the fundus oculi observation device 1 of the above embodiment has both a function of forming a tomographic image of the fundus oculi and a function of capturing a fundus oculi image, the device can also employ a configuration having only one of these functions.

For example, in the configuration having only the function of forming a tomographic image of the fundus oculi, a part (an accepting part) to accept a fundus oculi image captured by an external device is additionally installed.

An example of the accepting part is a network adapter that controls data communication with an external device. This accepting part is configured to be capable of communicating with an image database or a retinal camera, for example. In the image database, a fundus oculi image captured by the retinal camera or the like is stored.

The accepting part accesses the image database and acquires the fundus oculi image via a network. Moreover, in the case of accepting a fundus oculi image directly from the retinal camera or the like, the accepting part receives the fundus oculi image transmitted from the retinal camera or the like via the network.

As another example of the accepting part, it is possible to apply a reader (a drive or the like) that reads information recorded in a recording medium. The recording medium is, for example, an optical disk, a magneto-optical disk, and a magnetic recording medium, which will be described later. In the recording medium, a fundus oculi image captured by the retinal camera or the like is recorded in advance. The accepting part reads this fundus oculi image from the recording medium and inputs the image into the fundus oculi observation device.

This fundus oculi observation device, as in the above embodiment, has a first specifying part configured to specify a vascular region in a tomographic image, and a second specifying part configured to specify a vascular region in a fundus oculi image.

Moreover, this fundus oculi observation device has an image processor configured to obtain a common region of a vascular region in a tomographic image and a vascular region in a fundus oculi image, and to specify a region in the tomographic image corresponding to the common region. Furthermore, this fundus oculi observation device is provided with a display, and a controller configured to control the display to display the tomographic image so that a region corresponding to the common region can be visually recognized.

According to such a fundus oculi observation device, as in the above embodiment, it is possible to specify, of a vascular region in a tomographic image, a region common to a vascular region of a fundus oculi image, and therefore, it is possible to specify a vascular region in a tomographic image with higher accuracy than before based on both the images. Moreover, since it is possible to display a tomographic image so that a region corresponding to the common region can be visually recognized, it is possible to present the position of the vascular region in the tomographic image with high accuracy.

On the other hand, in the configuration having only the function of forming a fundus oculi image, a part (an accepting part) to accept a tomographic image captured by an external device is additionally disposed. The accepting part is configured by a network adapter and a reader as in the above example.

This fundus oculi observation device, as in the above embodiment, is provided with a first specifying part configured to specify a vascular region in a tomographic image, a second specifying part configured to specify a vascular region in a fundus oculi image, an image processor configured to obtain a common region to the vascular region in the tomographic image and the vascular region in the fundus oculi image and specify a region in the tomographic image corresponding to the common region, a display, and a controller configured to control the display to display the tomographic image so that the region corresponding to the common region can be visually recognized.

According to such a fundus oculi observation device, it is possible to specify, of the vascular region in the tomographic image, a region common to the vascular region in the fundus oculi image as in the above embodiment, and therefore, it is possible to specify a vascular region in a tomographic image with higher accuracy than before based on both the images. Moreover, since it is possible to display a tomographic image so that a region corresponding to the common region can be visually recognized, it is possible to present the position of the vascular region in the tomographic image with high accuracy.

### [Modification 2]

The fundus oculi observation device 1 of the above embodiment is configured to erase a vascular region common with the fundus oculi image Ef' from a tomographic image and add an image of a new layer position (an image of the estimated layer position) to the region.

However, there is no need to erase the vascular region.

For example, it is possible to superimpose an image of a new layer position on the vascular region. In this case, it is desirable to show so that the image of the new layer position is easy to see.

As a method for obtaining the new layer position, it is possible to apply the same method as in the above embodiment. Moreover, it is possible to apply the configuration of this modification to the modification 1. Moreover, as in the above embodiment, it is possible to dispose the configuration of obtaining the layer thickness of the fundus oculi.

### [Another Modification]

Although the position of the reference mirror 174 is changed and the difference in optical path length between the optical path of the signal light LS and the optical path of the reference light LR is changed in the above embodiment, the method for changing the difference in optical path length is not limited thereto. For example, it is possible to change the difference in optical path length by integrally moving the retinal camera unit 1A and the OCT unit 150 with respect to the eye E and changing the optical path length of the signal light LS.

Moreover, particularly in a case that a measured object is not a living body, it is also possible to change the difference in optical path length by moving the measured object in the depth direction (the z-direction).

### [FUNDUS OCULI IMAGE PROCESSING DEVICE]

An embodiment of a fundus oculi image processing device according to the present invention will be described.

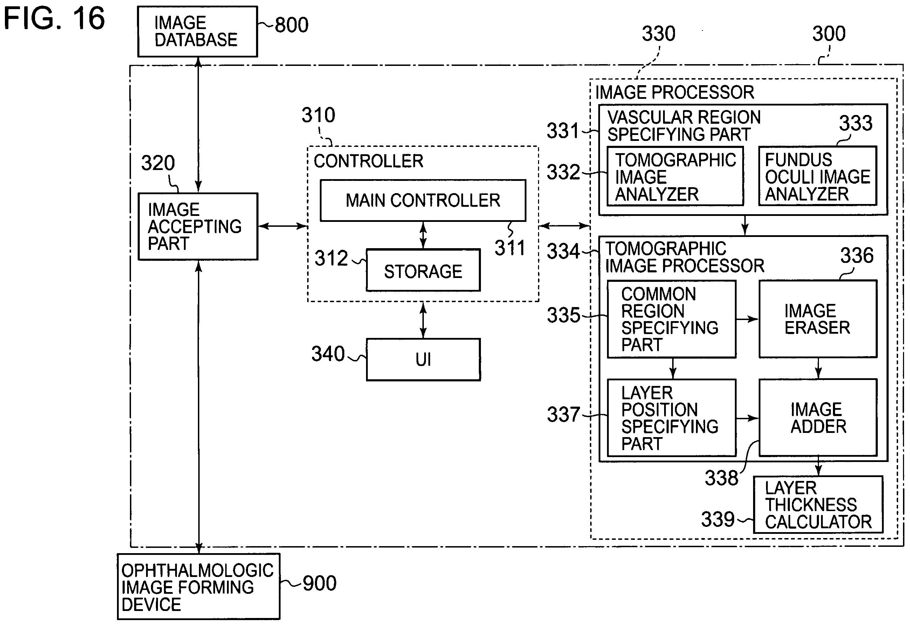

An example of the fundus oculi image processing device is shown in Fig. 16. A fundus oculi image processing device 300 is connected to an image database 800 and an ophthalmologic image forming device 900 so as to be communicable therewith via a communication line such as a LAN.

The image database 800 stores and manages various kinds of images in at least the ophthalmologic field. The image database 800 is in conformity with the DICOM (Digital Imaging and Communications in Medicine) standard, for example. A specific example of the image database 800 is a medical image filing system such as the PACS (Picture Archiving and Communications System), an electronic chart system, or the like. The image database 800, in response to a request from the fundus oculi image processing device 300, delivers an image.

The image database of the above modification is similar to this image database 800.

An ophthalmologic image forming device 900 is a generic name of various kinds of image forming devices used in the ophthalmologic field. The ophthalmologic image forming device 900 specifically forms an image of the fundus oculi. A specific example of the ophthalmologic image forming device 900 is, for example, an optical image measurement device (an OCT device) that forms a tomographic image and a three-dimensional image of the fundus oculi, and a retinal camera that captures a two-dimensional image of the fundus oculi surface. The ophthalmologic image forming device 900 transmits a formed image to the fundus oculi image processing device 300. In this process, the ophthalmologic image forming device 900 may once store a formed image and transmit the image in response to a request from the fundus oculi image processing device 300, or may transmit an image regardless of the presence of the request. Moreover, the ophthalmologic image forming device 900 may be connected to the image database 800 via a communication line. In this case, the fundus oculi image processing device 300 can receive images formed by the ophthalmologic image forming device 900 via the image database 800.

The fundus oculi image processing device 300 is configured by a general-purpose computer, for example, and has almost the same configuration in the arithmetic and control unit 200 of the above embodiment.

The fundus oculi image processing device 300 is provided with a controller 310 similar to the controller 210 of the arithmetic and control unit 200. The controller 310 is provided with a main controller 311 and a storage 312. The main controller 311 and the storage 312 are configured in the same manner as the main controller 211 and the storage 212, respectively, and execute the same operations. The main controller 311 is an example of the "controller" of the present invention.

The image accepting part 320 executes data communication with the image database 800 and the ophthalmologic image forming device 900 via the abovementioned communication line. The image accepting part 320 includes a network adapter such as a LAN card.

The image accepting part 320 may be a reader such as a drive that reads information recorded in a recording medium. In this case, the fundus oculi image processing device 300 does not need to be connected to the image database 800 or the ophthalmologic image forming part 900 via a communication line. The recording medium is, for example, an optical disk, a magneto-optical disk, a magnetic recording medium, or the like, which will be described later. Into a recording medium, an image stored in the image database 800 and an image formed by the ophthalmologic image processing device 900 are recorded. The image accepting part 320 reads the image recorded in the recording medium. The image accepting part 320 reads the image recorded in the recording medium and transmits to the controller 310.

The image accepting part 320 is an example of the "accepting part" of the present invention.

An image processor 330 has the same function as the image processor 230 of the arithmetic and control unit 200. The image processor 330 is provided with a vascular region specifying part 331, which is the same as the vascular region specifying part 231. The vascular region specifying part 331 is provided with a tomographic image analyzer 332 and a fundus oculi image analyzer 333. The tomographic image analyzer 332 is an example of the "first specifying part" of the present invention, and specifies a vascular region in a tomographic image of the fundus oculi in the same manner as the tomographic image analyzer 232 of the arithmetic and control unit 200.

The fundus oculi image analyzer 333 is an example of the "second specifying part" of the present invention, and specifies a vascular region in a two-dimensional image of the fundus oculi surface (a fundus oculi image) in the same manner as the fundus oculi image analyzer 233 of the arithmetic and control unit 200.

A tomographic image processor 334 is an example of the "image processor" of the present invention, and has the same function as the tomographic image processor 234 of the arithmetic and control unit 200. The tomographic image processor 334 is provided with a common region specifying part 335, an image eraser 336, a layer position specifying part 337, and an image adder 338.

The common region specifying part 335 specifies, of a vascular region in a tomographic image, a region (a common region) common to a vascular region in a fundus oculi image, in the same manner as the common region specifying part 235 of the arithmetic and control unit 200. The image eraser 336 erases an image of the common region from the tomographic image, in the same manner as the image eraser 236 of the arithmetic and control unit 200. The layer position specifying part 337 analyzes the tomographic image and specifies the layer position (the boundary position of the layers) of the fundus oculi, in the same manner as the layer position specifying part 237 of the arithmetic and control unit 200. To be specific, the layer position specifying part 337 estimates the layer position within the common region based on the state of the layer position in the neighborhood of the common region.

The image adder 338 adds an image showing the estimated layer position to the common region (a region from which the image has been erased) in the tomographic image, in the same manner as the image adder 238 of the arithmetic and control unit 200.

A layer thickness calculator 339 calculates the layer thickness of the fundus oculi based on the tomographic image, in the same manner as the layer thickness calculator 239 of the arithmetic and control unit 200. To be specific, the layer thickness calculator 339 calculates the layer thickness based on the image of the layer position, for the common region to which the image showing the layer position has been added.

A user interface (UI) 340 is used as a console of the fundus oculi image processing device 300, and includes a display device, a manipulation device and an input device as the user interface 240 of the arithmetic and control unit 200 does. The display device (the same as the display 240A of the above embodiment) is an example of the "display" of the present invention.

The main controller 311 controls the display device to display various kinds of information such as the tomographic image or fundus oculi image accepted by the image accepting part 320, the tomographic image or fundus oculi image in which the vascular region is enhanced, the tomographic image in which the common region is enhanced, the tomographic image in which the common region is erased, the tomographic image in which the layer position is enhanced, and the result of calculation of the layer thickness (the layer thickness graph, the layer thickness distribution image, or the like).

According to the fundus oculi image processing device 300, it is possible to specify, of the vascular region in the tomographic image, a region common to the vascular region in the fundus oculi image.

Therefore, it is possible to specify the vascular region in the tomographic image with higher accuracy than before based on both the images.

Further, in the case of forming a three-dimensional image from a plurality of tomographic images, by executing the process on the respective tomographic images, it is possible to specify the vascular region in the three-dimensional image with higher accuracy.

Further, according to the fundus oculi image processing device 300, it is possible to obtain the layer position in the vascular region common with the fundus oculi image based on the layer position in the neighborhood thereof. Therefore, it is possible to obtain the layer position of the vascular region with higher accuracy. Furthermore, since the device acts to obtain the layer thickness in the vascular region based on the thus obtained layer position, it is possible to obtain the layer thickness in the vascular region with higher accuracy.

The various kinds of configurations and operations described in the above embodiment and the above modification as examples of the fundus oculi observation device according to the present invention can be properly added to the fundus oculi image processing device 300.

### [PROGRAM]

A program according to the present invention is a program for controlling a computer that stores a tomographic image of the fundus oculi and a two-dimensional image of the fundus oculi surface (a fundus oculi image). This computer shall be provided with a display.

The control program 204a of the above embodiment is an example of the program according to the present invention.

A program according to the present invention causes the computer to function as the following parts: (1) a first specifying part configured to specify a vascular region in the tomographic image of the fundus oculi; (2) a second specifying part configured to specify a vascular region in the fundus oculi image; (3) an image processor configured to obtain a common region of the vascular region in the tomographic image and the vascular region in the two-dimensional image; and (4) a controller configured to control a display to display the tomographic image so that the region corresponding to the common region can be visually recognized.

According to the computer controlled by this program, it is possible to specify, of a vascular region in a tomographic image, a vascular region common with a fundus oculi image. Therefore, it is possible to specify a vascular region in a tomographic image with higher accuracy than before based on both the images.

The program according to the present invention can be stored into an arbitrary recording medium that can be read by a driver of the computer. Such a recording medium is, for example, an optical disk, a magneto-optical disk (CD-ROM, DVD-RAM ,DVD-ROM ,MO, and so on), a magnetic recording medium (a hard disk, a floppy^{™} disk, ZIP, and so on), and a USB memory. Moreover, it is possible to store into a storing device installed in the computer, such as a hard disk drive and a memory. Furthermore, it is possible to transmit this program through a network such as the Internet and LAN.

## Claims

1. A fundus oculi observation device (1), comprising:
an acquiring part configured to acquire a tomographic image of a fundus oculi (Ef) and a two-dimensional image (Ef') of a surface of the fundus oculi (Ef);
a first specifying part (232) configured to analyze the tomographic image to specify a vascular region in the tomographic image;
a second specifying part (233) configured to analyze the two-dimensional image (Ef') to specify a vascular region in the two-dimensional image (Ef');
an image processor (234) configured to obtain a common region of the vascular region in the tomographic image and the vascular region in the two-dimensional image (Ef'), and specify a region in the tomographic image corresponding to the common region;
a display (240A); and
a controller (211) configured to control the display (240A) to display the tomographic image so that the region corresponding to the common region can be visually recognized.

2. The fundus oculi observation device according to Claim 1, wherein the acquiring part includes: a part configured to split a low-coherence light (L0) into a signal light (LS) and a reference light (LR), superimpose the signal light (LS) propagated through the fundus oculi (Ef) and the reference light (LR) propagated through a reference object (174) to generate an interference light (LC), and detect the interference light (LC) to form the tomographic image of the fundus oculi (Ef); and an imaging part (208a) configured to radiate an illumination light to the fundus oculi (Ef), and detect a fundus oculi reflected light of the illumination light to capture the two-dimensional image (Ef') of the surface of the fundus oculi (Ef).

3. The fundus oculi observation device according to Claim 1, wherein the acquiring part includes: a part configured to split a low-coherence light (L0) into a signal light (LS) and a reference light (LR), superimpose the signal light (LS) propagated through the fundus oculi (Ef) and the reference light (LR) propagated through a reference object (174) to generate an interference light (LC), and detect the interference light (LC) to form the tomographic image of the fundus oculi (Ef); and an accepting part (320) configured to accept the two-dimensional image (Ef') of the surface of the fundus oculi (Ef).

4. The fundus oculi observation device according to Claim 1, wherein the acquiring part includes: an accepting part (320) configured to accept the tomographic image of the fundus oculi (Ef); and an imaging part (208a) configured to radiate an illumination light to the fundus oculi (Ef), and detect a fundus oculi reflected light of the illumination light to capture the two-dimensional image (Ef') of the surface of the fundus oculi (Ef).

5. The fundus oculi observation device according to Claim 1, wherein the image processor (234) is configured to erase an image of the region in the tomographic image corresponding to the common region.

6. The fundus oculi observation device according to Claim 5, wherein the image processor (234) is configured to analyze the tomographic image to specify a layer position of the fundus oculi (Ef) in a neighborhood region of the common region, and add an image representing the layer position to the region corresponding to the common region, based on the layer position in the neighborhood region.

7. The fundus oculi observation device according to Claim 1, wherein the image processor (234) is configured to analyze the tomographic image to specify a layer position of the fundus oculi (Ef) in a neighborhood region of the common region, and add an image representing the layer position to the region in the tomographic image corresponding to the common region, based on the layer position in the neighborhood region.

8. The fundus oculi observation device according to Claim 6, wherein the image processor (234) is configured to specify a boundary region of a layer as the layer position based on pixel values of pixels in the neighborhood region, estimate a boundary position of the layer in the common region based on a morphology of the boundary region, and add an image representing the estimated boundary position as an image representing the layer position.

9. The fundus oculi observation device according to Claim 7, wherein the image processor (234) is configured to specify a boundary region of a layer as the layer position based on pixel values of pixels in the neighborhood region, estimate a boundary position of the layer in the common region based on a morphology of the boundary region, and add an image representing the estimated boundary position as an image representing the layer position.

10. The fundus oculi observation device according to Claim 8, wherein the image processor (234) is configured to, for each of the neighborhood regions on both sides of the common region, obtain a position of the boundary region of the layer at a boundary between the neighborhood region and the common region, estimate a position on a straight line connecting positions on both the sides as the boundary position, and add the line as the image representing the boundary position.

11. The fundus oculi observation device according to Claim 9, wherein the image processor (234) is configured to, for each of the neighborhood regions on both sides of the common region, obtain a position of the boundary region of the layer at a boundary between the neighborhood region and the common region, estimate a position on a straight line connecting positions on both the sides as the boundary position, and add the line as the image representing the boundary position.

12. The fundus oculi observation device according to Claim 8, wherein the image processor (234) is configured to, for each of the neighborhood regions on both sides of the common region, obtain a position and slope of the boundary region of the layer at a boundary between the neighborhood region and the common region, estimate a position on a spline curve connecting positions on both the sides as the boundary position based on the position and slope, and add the spline curve as an image representing the boundary position.

13. The fundus oculi observation device according to Claim 9, wherein the image processor (234) is configured to, for each of the neighborhood regions on both sides of the common region, obtain a position and slope of the boundary region of the layer at a boundary between the neighborhood region and the common region, estimate a position on a spline curve connecting positions on both the sides as the boundary position based on the position and slope, and add the spline curve as an image representing the boundary position.

14. The fundus oculi observation device according to Claim 6, comprising a calculator configured to calculate a layer thickness of the fundus oculi (Ef) in the common region, based on the image representing the layer position.

15. The fundus oculi observation device according to Claim 7, comprising a calculator (239) configured to calculate a layer thickness of the fundus oculi (Ef) in the common region, based on the image representing the layer position.

16. A fundus oculi observation method for analyzing a tomographic image of a fundus oculi (Ef) and a two-dimensional image (Ef') of a surface of the fundus oculi (Ef), comprising steps of:
analyzing the tomographic image to specify a vascular region in the tomographic image;
analyzing the two-dimensional image (Ef') to specify a vascular region in the two-dimensional image (Ef');
obtaining a common region of the vascular region in the tomographic image and the vascular region in the two-dimensional image (Ef');
specifying a region in the tomographic image corresponding to the common region; and
displaying the tomographic image so that the region corresponding to the common region can be visually recognized.

## Patentansprüche

1. Augenhintergrundbeobachtungsvorrichtung (1), umfassend:
einen Erlangungsteil, der konfiguriert ist, ein Schichtbild eines Augenhintergrunds (Ef) und ein zweidimensionales Bild (Ef') einer Oberfläche des Augenhintergrunds (Ef) zu erlangen;
einen ersten Spezifizierungsteil (232), der konfiguriert ist, das Schichtbild zu analysieren, um eine Gefäßregion in dem Schichtbild zu spezifizieren;
einen zweiten Spezifizierungsteil (233), der konfiguriert ist, das zweidimensionale Bild (Ef') zu analysieren, um eine Gefäßregion in dem zweidimensionalen Bild (Ef') zu spezifizieren;
einen Bildprozessor (234), der konfiguriert ist, eine gemeinsame Region der Gefäßregion in dem Schichtbild und der Gefäßregion in dem zweidimensionalen Bild (Ef') zu erhalten, und eine Region in dem Schichtbild zu spezifizieren, die der gemeinsamen Region entspricht;
eine Anzeige (240A); und
eine Steuerung (211), die konfiguriert ist, die Anzeige (240A) zu steuern, um das Schichtbild anzuzeigen, sodass die Region, die der gemeinsamen Region entspricht, visuell erkannt werden kann.

2. Augenhintergrundbeobachtungsvorrichtung nach Anspruch 1, wobei der Erlangungsteil umfasst: einen Teil, der konfiguriert ist, ein Licht mit niedriger Kohärenz (L0) in ein Signallicht (LS) und ein Bezugslicht (LR) aufzuteilen, das Signallicht (LS), das durch den Augenhintergrund (Ef) propagiert ist, und das Bezugslicht (LR), das durch ein Bezugsobjekt (174) propagiert ist, zu überlagern, um ein Interferenzlicht (LC) zu erzeugen, und das Interferenzlicht (LC) zu detektieren, um das Schichtbild des Augenhintergrunds (Ef) zu bilden; und einen Bildgebungsteil (208a), der konfiguriert ist, ein Beleuchtungslicht auf den Augenhintergrund (Ef) zu strahlen und ein von dem Augenhintergrund reflektiertes Licht des Beleuchtungslichts zu detektieren, um das zweidimensionale Bild (Ef') der Oberfläche des Augenhintergrunds (Ef) zu erfassen.

3. Augenhintergrundbeobachtungsvorrichtung nach Anspruch 1, wobei der Erlangungsteil umfasst: einen Teil, der konfiguriert ist, ein Licht mit niedriger Kohärenz (L0) in ein Signallicht (LS) und ein Bezugslicht (LR) aufzuteilen, das Signallicht (LS), das durch den Augenhintergrund (Ef) propagiert ist, und das Bezugslicht (LR), das durch ein Bezugsobjekt (174) propagiert ist, zu überlagern, um ein Interferenzlicht (LC) zu erzeugen, und das Interferenzlicht (LC) zu detektieren, um das Schichtbild des Augenhintergrunds (Ef) zu bilden; und einen Annahmeteil (320), der konfiguriert ist, das zweidimensionale Bild (Ef') der Oberfläche des Augenhintergrunds (Ef) anzunehmen.

4. Augenhintergrundbeobachtungsvorrichtung nach Anspruch 1, wobei der Erlangungsteil umfasst: einen Annahmeteil (320), der konfiguriert ist, das Schichtbild des Augenhintergrunds (Ef) anzunehmen; und einen Bildgebungsteil (208a), der konfiguriert ist, ein Beleuchtungslicht auf den Augenhintergrund (Ef) zu strahlen und ein von dem Augenhintergrund reflektiertes Licht des Beleuchtungslichts zu detektieren, um das zweidimensionale Bild (Ef') der Oberfläche des Augenhintergrunds (Ef) zu erfassen.

5. Augenhintergrundbeobachtungsvorrichtung nach Anspruch 1, wobei der Bildprozessor (234) konfiguriert ist, ein Bild der Region in dem Schichtbild, die der gemeinsamen Region entspricht, zu löschen.

6. Augenhintergrundbeobachtungsvorrichtung nach Anspruch 5, wobei der Bildprozessor (234) konfiguriert ist, das Schichtbild zu analysieren, um eine Schichtposition des Augenhintergrunds (Ef) in einer Nachbarschaftsregion der gemeinsamen Region zu spezifizieren, und ein Bild, das die Schichtposition darstellt, zu der Region, die der gemeinsamen Region entspricht, basierend auf der Schichtposition in der Nachbarschaftsregion hinzuzufügen.

7. Augenhintergrundbeobachtungsvorrichtung nach Anspruch 1, wobei der Bildprozessor (234) konfiguriert ist, das Schichtbild zu analysieren, um eine Schichtposition des Augenhintergrunds (Ef) in einer Nachbarschaftsregion der gemeinsamen Region zu spezifizieren, und ein Bild, das die Schichtposition darstellt, zu der Region in dem Schichtbild, die der gemeinsamen Region entspricht, basierend auf der Schichtposition in der Nachbarschaftsregion hinzuzufügen.

8. Augenhintergrundbeobachtungsvorrichtung nach Anspruch 6, wobei der Bildprozessor (234) konfiguriert ist, eine Grenzregion einer Schicht basierend auf Pixelwerten von Pixeln in der Nachbarschaftsregion als die Schichtposition zu spezifizieren, eine Grenzposition der Schicht in der gemeinsamen Region basierend auf einer Morphologie der Grenzregion abzuschätzen und ein Bild, das die abgeschätzte Grenzposition darstellt, als ein Bild hinzuzufügen, das die Schichtposition darstellt.

9. Augenhintergrundbeobachtungsvorrichtung nach Anspruch 7, wobei der Bildprozessor (234) konfiguriert ist, eine Grenzregion einer Schicht basierend auf Pixelwerten von Pixeln in der Nachbarschaftsregion als die Schichtposition zu spezifizieren, eine Grenzposition der Schicht in der gemeinsamen Region basierend auf einer Morphologie der Grenzregion abzuschätzen und ein Bild, das die abgeschätzte Grenzposition darstellt, als ein Bild hinzuzufügen, das die Schichtposition darstellt.

10. Augenhintergrundbeobachtungsvorrichtung nach Anspruch 8, wobei der Bildprozessor (234) konfiguriert ist, für jede der Nachbarschaftsregionen auf beiden Seiten der gemeinsamen Region eine Position der Grenzregion der Schicht an einer Grenze zwischen der Nachbarschaftsregion und der gemeinsamen Region zu erhalten, eine Position auf einer geraden Linie, die Positionen auf beiden Seiten verbindet, als die Grenzposition abzuschätzen und die Linie als das Bild, das die Grenzposition darstellt, hinzuzufügen.

11. Augenhintergrundbeobachtungsvorrichtung nach Anspruch 9, wobei der Bildprozessor (234) konfiguriert ist, für jede der Nachbarschaftsregionen auf beiden Seiten der gemeinsamen Region eine Position der Grenzregion der Schicht an einer Grenze zwischen der Nachbarschaftsregion und der gemeinsamen Region zu erhalten, eine Position auf einer geraden Linie, die Positionen auf beiden Seiten verbindet, als die Grenzposition abzuschätzen und die Linie als das Bild, das die Grenzposition darstellt, hinzuzufügen.

12. Augenhintergrundbeobachtungsvorrichtung nach Anspruch 8, wobei der Bildprozessor (234) konfiguriert ist, für jede der Nachbarschaftsregionen auf beiden Seiten der gemeinsamen Region eine Position und eine Neigung der Grenzregion der Schicht an einer Grenze zwischen der Nachbarschaftsregion und der gemeinsamen Region zu erhalten, eine Position auf einer Spline-Kurve, die Positionen auf beiden Seiten verbindet, basierend auf der Position und der Neigung als die Grenzposition abzuschätzen und die Spline-Kurve als ein Bild hinzuzufügen, das die Grenzposition darstellt.

13. Augenhintergrundbeobachtungsvorrichtung nach Anspruch 9, wobei der Bildprozessor (234) konfiguriert ist, für jede der Nachbarschaftsregionen auf beiden Seiten der gemeinsamen Region eine Position und eine Neigung der Grenzregion der Schicht an einer Grenze zwischen der Nachbarschaftsregion und der gemeinsamen Region zu erhalten, eine Position auf einer Spline-Kurve, die Positionen auf beiden Seiten verbindet, basierend auf der Position und der Neigung als die Grenzposition abzuschätzen und die Spline-Kurve als ein Bild hinzuzufügen, das die Grenzposition darstellt.

14. Augenhintergrundbeobachtungsvorrichtung nach Anspruch 6, umfassend eine Rechenvorrichtung, die konfiguriert ist, eine Schichtdicke des Augenhintergrunds (Ef) in der gemeinsamen Region basierend auf dem Bild, das die Schichtposition darstellt, zu berechnen.

15. Augenhintergrundbeobachtungsvorrichtung nach Anspruch 7, umfassend eine Rechenvorrichtung (239), die konfiguriert ist, eine Schichtdicke des Augenhintergrunds (Ef) in der gemeinsamen Region basierend auf dem Bild, das die Schichtposition darstellt, zu berechnen.

16. Augenhintergrundbeobachtungsverfahren zum Analysieren eines Schichtbildes eines Augenhintergrunds (Ef) und eines zweidimensionalen Bildes (Ef') einer Oberfläche des Augenhintergrunds (Ef), umfassend die Schritte:
Analysieren des Schichtbildes, um eine Gefäßregion in dem Schichtbild zu spezifizieren;
Analysieren des zweidimensionalen Bildes (Ef'), um eine Gefäßregion in dem zweidimensionalen Bild (Ef') zu bestimmen;
Erhalten einer gemeinsamen Region der Gefäßregion in dem Schichtbild und der Gefäßregion in dem zweidimensionalen Bild (Ef');
Spezifizieren einer Region in dem Schichtbild, die der gemeinsamen Region entspricht; und
Anzeigen des Schichtbildes, sodass die Region, die der gemeinsamen Region entspricht, visuell erkannt werden kann.

## Revendications

1. Dispositif d'observation de fond d'œil (1) comprenant :
une partie d'acquisition configurée pour acquérir une image tomographique d'un fond d'œil (Ef) et une image bidimensionnelle (Ef') d'une surface d'un fond d'œil (Ef) ;
une première partie de spécification (232) configurée pour analyser l'image tomographique pour spécifier une zone de vaisseaux sanguins dans l'image tomographique ;
une deuxième partie de spécification (233) configurée pour analyser l'image bidimensionnelle (Ef') pour spécifier une zone de vaisseaux sanguins dans l'image bidimensionnelle (Ef') ;
un processeur d'image (234) configuré pour obtenir une zone commune de la zone de vaisseaux sanguins dans l'image tomographique et de la zone de vaisseaux sanguins dans l'image bidimensionnelle (Ef'), et spécifier une zone dans l'image tomographique correspondant à la zone commune ;
un afficheur (240A) ; et
un contrôleur (211) configuré pour contrôler l'afficheur (240A) pour afficher l'image tomographique de sorte que la zone correspondant à la zone commune peut être reconnue visuellement.

2. Dispositif d'observation de fond d'œil selon la revendication 1, dans lequel la partie d'acquisition comprend : une partie configurée pour séparer de la lumière à faible cohérence (L0) en une lumière de signalisation (LS) et une lumière de référence (LR), superposer la lumière de signalisation (LS) qui s'est propagée à travers le fond d'œil (Ef) et la lumière de référence (LR) qui s'est propagée à travers un objet de référence (174) pour générer une lumière d'interférence (LC), et détecter la lumière d'interférence (LC) pour former l'image tomographique du fond d'œil (Ef) ; et une partie d'imagerie (208a) configurée pour faire rayonner une lumière d'illumination vers le fond d'œil (Ef) et détecter une lumière réfléchie par le fond d'œil de la lumière d'illumination pour capturer l'image bidimensionnelle (Ef') de la surface du fond d'œil (Ef).

3. Dispositif d'observation de fond d'œil selon la revendication 1, dans lequel la partie d'acquisition comprend : une partie configurée pour séparer une lumière à faible cohérence (L0) en une lumière de signalisation (LS) et une lumière de référence (LR), superposer la lumière de signalisation (LS) qui s'est propagée à travers le fond d'œil (Ef) et la lumière de référence (LR) qui s'est propagée à travers un objet de référence (174) pour générer une lumière d'interférence (LC), et détecter la lumière d'interférence (LC) pour former l'image tomographique du fond d'œil (Ef) ; et une partie d'acceptation (320) configurée pour accepter l'image bidimensionnelle (Ef') de la surface du fond d'œil (Ef).

4. Dispositif d'observation de fond d'œil selon la revendication 1, dans lequel la partie d'acquisition comprend : une partie d'acceptation (320) configurée pour accepter l'image tomographique du fond d'œil (Ef) ; et une partie d'imagerie (208a) configurée pour faire rayonner une lumière d'illumination vers le fond d'œil (Ef) et détecter une lumière réfléchie par le fond d'œil de la lumière d'illumination pour capturer l'image bidimensionnelle (Ef') de la surface du fond d'œil (Ef).

5. Dispositif d'observation de fond d'œil selon la revendication 1, dans lequel le processeur d'image (234) est configuré pour effacer une image de la zone dans l'image tomographique correspondant à la zone commune.

6. Dispositif d'observation de fond d'œil selon la revendication 5, dans lequel le processeur d'image (234) est configuré pour analyser l'image tomographique pour spécifier une position de couche du fond d'œil (Ef) dans une zone de voisinage de la zone commune, et ajouter une image représentant la position de couche à la zone correspondant à la zone commune sur la base de la position de couche dans la zone de voisinage.

7. Dispositif d'observation de fond d'œil selon la revendication 1, dans lequel le processeur d'image (234) est configuré pour analyser l'image tomographique pour spécifier une position de couche du fond d'œil (Ef) dans une zone de voisinage de la zone commune, et ajouter une image représentant la position de couche à la zone dans l'image tomographique correspondant à la zone commune sur la base de la position de couche dans la zone de voisinage.

8. Dispositif d'observation de fond d'œil selon la revendication 6, dans lequel le processeur d'image (234) est configuré pour spécifier une zone limitrophe d'une couche en tant que position de couche sur la base de valeurs de pixel de pixels dans la zone de voisinage, estimer une position limitrophe de la couche dans la zone commune sur la base d'une morphologie de la zone limitrophe, et ajouter une image représentant la position limitrophe estimée en tant qu'image représentant la position de couche.

9. Dispositif d'observation de fond d'œil selon la revendication 7, dans lequel le processeur d'image (234) est configuré pour spécifier une zone limitrophe d'une couche en tant que position de couche sur la base de valeurs de pixels de pixels dans la zone de voisinage, estimer une position limitrophe de la couche dans la zone commune sur la base d'une morphologie de la zone limitrophe, et ajouter une image représentant la position limitrophe estimée en tant qu'image représentant la position de couche.

10. Dispositif d'observation de fond d'œil selon la revendication 8, dans lequel le processeur d'image (234) est configuré pour, pour chacune des zones de voisinage des deux côtés de la zone commune, obtenir une position de la zone limitrophe de la couche à une limite entre la zone de voisinage et la zone commune, estimer une position sur une ligne droite reliant des positions des deux côtés en tant que position limitrophe, et ajouter la ligne en tant qu'image représentant la position limitrophe.

11. Dispositif d'observation de fond d'œil selon la revendication 9, dans lequel le processeur d'image (234) est configuré pour, pour chacune des zones de voisinage des deux côtés de la zone commune, obtenir une position de la zone limitrophe de la couche à une limite entre la zone de voisinage et la zone commune, estimer une position sur une ligne droite reliant des positions des deux côtés en tant que position limitrophe, et ajouter la ligne en tant qu'image représentant la position limitrophe.

12. Dispositif d'observation de fond d'œil selon la revendication 8, dans lequel le processeur d'image (234) est configuré pour, pour chacune des zones de voisinage des deux côtés de la zone commune, obtenir une position et une pente de la zone limitrophe de la couche à une limite entre la zone de voisinage et la zone commune, estimer une position sur une courbe spline reliant des positions des deux côtés en tant que position limitrophe sur la base de la position et pente, et ajouter la courbe spline en tant qu'image représentant la position limitrophe.

13. Dispositif d'observation de fond d'œil selon la revendication 9, dans lequel le processeur d'image (234) est configuré pour, pour chacune de zones de voisinage des deux côtés de la zone commune, obtenir une position et pente de la zone limitrophe de la couche à une limite entre la zone de voisinage et la zone commune, estimer une position sur une courbe spline reliant des positions des deux côtés en tant que position limitrophe sur la base de la position et pente, et ajouter la courbe spline en tant qu'image représentant la position limitrophe.

14. Dispositif d'observation de fond d'œil selon la revendication 6, comprenant un calculateur configuré pour calculer une épaisseur de couche du fond d'œil (Ef) dans la zone commune sur la base de l'image représentant la position de couche.

15. Dispositif d'observation de fond d'œil selon la revendication 7, comprenant un calculateur (239) configuré pour calculer une épaisseur de couche du fond d'œil (Ef) dans la zone commune sur la base de l'image représentant la position de couche.

16. Procédé d'observation de fond d'œil pour analyser une image tomographique d'un fond d'œil (Ef) et une image bidimensionnelle (Ef') d'une surface du fond d'œil (Ef), comprenant les étapes suivantes :
l'analyse de l'image tomographique pour spécifier une zone de vaisseaux sanguins dans l'image tomographique ;
l'analyse de l'image bidimensionnelle (Ef') pour spécifier une zone de vaisseaux sanguins dans l'image bidimensionnelle (Ef') ;
l'obtention d'une zone commune de la zone de vaisseaux sanguins dans l'image tomographique et de la zone de vaisseaux sanguins dans l'image bidimensionnelle (Ef') ;
la spécification d'une zone dans l'image tomographique correspondant à la zone commune ; et
l'affichage de l'image tomographique de sorte que la zone correspondant à la zone commune peut être reconnue visuellement.
